(19) Europäisches Patentamt
European Patent Office
Office européen des brevets

(11) Veröffentlichungsnummer: **0 433 879 A2**

# (12) EUROPÄISCHE PATENTANMELDUNG

(21) Anmeldenummer: **90124027.5**

(22) Anmeldetag: **13.12.90**

(51) Int. Cl.[5]: **C07D 277/38**, C07D 277/56, C07D 417/12

(30) Priorität: **22.12.89 DE 3942581**
**20.09.90 DE 4029732**

(43) Veröffentlichungstag der Anmeldung:
**26.06.91 Patentblatt 91/26**

(84) Benannte Vertragsstaaten:
**CH DE FR GB IT LI**

(71) Anmelder: **BASF Aktiengesellschaft**
**Carl-Bosch-Strasse 38**
**W-6700 Ludwigshafen(DE)**

(72) Erfinder: **Wiesenfeldt, Matthias, Dr.**
**Rosenstrasse 10**
**W-6704 Mutterstadt(DE)**
Erfinder: **Etzbach, Karl-Heinz, Dr.**
**Carl-Bosch-Ring 55**
**W-6710 Frankenthal(DE)**
Erfinder: **Gruettner, Sabine, Dr.**
**Neuweg 11**
**W-6704 Mutterstadt(DE)**
Erfinder: **Reichelt, Helmut, Dr.**
**Johann-Gottlieb-Fichte-Strasse 56**
**W-6730 Neustadt(DE)**

## (54) Aminothiazole.

(57) Aminothiazole der Formel

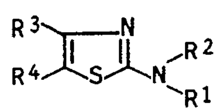

oder deren Tautomere, worin
$R^1$ und $R^2$ jeweils Wasserstoff oder zusammen einen Rest der Formel

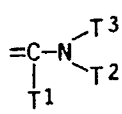 ,

in der $T^1$ für Wasserstoff, Alkyl oder Phenyl, $T^2$ und $T^3$ unabhängig voneinander für Alkyl oder Phenyl, oder $T^2$ und $T^3$ zusammen mit dem sie verbindenden Stickstoffatom für einen heterocyclischen Rest stehen,
$R^3$ substituiertes Mercapto oder substituiertes Amino und
$R^4$ Alkanoyl, Benzoyl, Cyano oder einen Rest der Formel

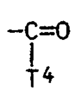 oder 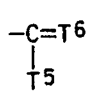

bedeuten, worin $T^4$ für Hydroxy, $C_1$-$C_6$-Alkoxy, Amino oder den Rest $R^3$, $T^5$ für Wasserstoff, Alkyl oder

EP 0 433 879 A2

Phenyl und $T^6$ für den Rest eines primären Amins oder einer methylenaktiven Verbindung stehen, sowie ein Verfahren zu ihrer Herstellung.

# AMINOTHIAZOLE

Die vorliegende Erfindung betrifft neue Aminothiazole der Formel I

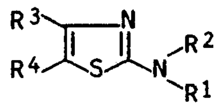

(I)

oder deren Tautomere, wobei

$R^1$ und $R^2$ jeweils Wasserstoff oder zusammen einen Rest der Formel

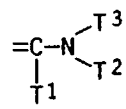

,

worin $T^1$ für Wasserstoff, $C_1$-$C_6$-Alkyl oder Phenyl und $T^2$ und $T^3$ gleich oder verschieden sind und unabhängig voneinander jeweils für $C_1$-$C_6$-Alkyl, $C_5$-$C_7$-Cycloalkyl oder Phenyl oder $T^2$ und $T^3$ zusammen mit dem sie verbindenden Stickstoffatom für einen 5- bis 7-gliedrigen gesättigten heterocyclischen Rest, der gegebenenfalls weitere Heteroatome enthält, stehen,

$R^3$ einen Rest der Formel

$$-S(O)_n-X \quad ,$$

worin n für 0, 1 oder 2 und x für gegebenenfalls substituiertes $C_1$-$C_{20}$-Alkyl, $C_3$-$C_8$-Cycloalkyl, gegebenenfalls substituiertes $c_3$-$c_6$-Alkenyl, gegebenenfalls substituiertes $C_3$-$C_6$-Alkinyl, gegebenenfalls substituiertes Phenyl oder, wenn n die Bedeutung von 0 besitzt, auch für Wasserstoff stehen, oder einen Rest der Formel Y, der die Bedeutung von $C_1$-$C_{20}$-Mono- oder Dialkylamino, worin die Alkylkette gegebenenfalls substituiert ist und durch ein oder mehrere Sauerstoffatome unterbrochen sein kann, $C_3$-$C_8$-Cycloalkylamino, Adamantylamino, $c_2$-$c_{12}$-Mono-oder Dialkenylamino, $C_3$-$C_{12}$-Alkinylamino, N-($C_1$-$C_5$-Alkyl)-N-phenylamino, Pyrrolidino, Piperidino, Morpholino, Thiomorpholino, Piperazino, N($C_1$-$C_4$-Alkyl)piperazino, Hexamethylenimino, Imidazol-1-yl, Pyrazol-1-yl, gegebenenfalls substituiertes Phenylamino, Pyridylamino, Thienylamino, Hydrazino, $C_1$-$C_4$-Mono- oder Dialkylhydrazino oder Phenylhydrazino besitzt, und

$R^4$ $C_1$-$C_6$-Alkanoyl, Benzoyl, Cyano oder einen Rest der Formel

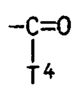 oder 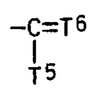

bedeuten, worin $T^4$ für Hydroxy, $C_1$-$C_6$-Alkoxy, Amino oder den obengenannten Rest Y,$T^5$ für Wasserstoff, $C_1$-$C_6$-Alkyl oder Phenyl und $T^6$ für den Rest einer methylenaktiven Verbindung, Hydroxyimino oder den Rest N-Q stehen, in dem Q die Bedeutung von $C_1$-$C_{20}$-Alkyl, das gegebenenfalls substituiert ist und durch ein oder mehrere Sauerstoffatome unterbrochen sein kann, gegebenenfalls substituiertem $C_3$-$C_6$-Alkenyl, gegebenenfalls substituiertem $C_3$-$C_6$-Alkinyl, gegebenenfalls substituiertem $C_3$-$C_{12}$-Cycloalkyl, gegebenenfalls substituiertem Phenyl, Pyridyl, $C_1$-$C_4$-Alkoxycarbonylmethyl, Amino, $C_1$-$C_4$-Dialkylamino oder Phenylamino besitzt,

mit der Maßgabe, daß

a) wenn $R^1$ und $R^2$ jeweils Wasserstoff und $R^4$ Acetyl oder Ethoxycarbonyl bedeuten, $R^3$ nicht für den Rest der Formel

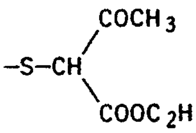

oder $-S-CH(COCH_{23})_2$

stehen,

b) wenn $R^1$ und $R^2$ jeweils Wasserstoff und $R^4$ Cyano bedeuten, $R^3$ nicht für Methylthio steht, und

c) wenn $R^3$ für Piperidino oder Morpholino und $R^4$ für Cyano stehen, $R^1$ und $R^2$ nicht gleichzeitig Wasserstoff bedeuten.

Aus Tetrahedron Lett. Band 22, Seiten 2285 bis 2288, 1982 ist bereits die Verbindung 2-Amino-4-methylthio-5-cyanothiazol bekannt. Weiterhin sind in Tetrahedron Lett. Band 28, Seiten 117 bis 120, 1987 2-Aminothiazole beschrieben, die in Ringposition 5 entweder die Acetyl- oder Ethoxycarbonylgruppe und in Ringposition 4 entweder den Rest

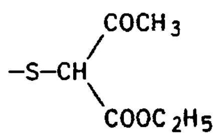

oder $-S-CH(COCH_3)_2$

aufweisen.

Es sind auch bereits 2,4-Diaminothiazolderivate bekannt, die in Ringposition 2 eine substituierte Aminogruppe, in Ringposition 4 eine Piperidino- oder Morpholinogruppe und in Ringposition 5 eine Cyanogruppe aufweisen (siehe z.B. Tetrahedron Lett. Band 22, Seiten 2285 bis 2288, 1981).

Die genannten Thiazole eignen sich jedoch nicht besonders gut als Diazokomponenten für die Herstellung von Azofarbstoffen.

Aufgabe der vorliegenden Erfindung war es nun, neue 2-Aminothiazole bereitzustellen, die in Ringposition 4 eine Thiolgruppe oder eine substituierte Aminogruppe aufweisen und sich gut als Diazokomponente oder deren Vorstufe für die Herstellung von Azofarbstoffen eignen sollten.

Demgemäß wurden die oben näher bezeichneten Aminothiazole der Formel I gefunden.

Alle in der obengenannten Formel I auftretenden Alkyl- und Alkenylgruppen können sowohl geradkettig als auch verzweigt sein.

Wenn in der Formel I Alkylgruppen auftreten, die durch ein oder mehrere Sauerstoffatome unterbrochen sind, sind jene bevorzugt, die durch ein bis drei, insbesondere ein bis zwei Sauerstoffatome unterbrochen sind.

Wenn in der Formel I substituierte Phenylgruppen auftreten, können als Substituenten z.B. $C_1$-$C_6$-Alkyl, $C_1$-$C_6$-Alkoxy, Halogen, dabei insbesondere Fluor, Chlor oder Brom, Nitro, Hydroxy, Amino, $C_1$-$C_6$-Mono- oder Dialkylamino, Acetylamino, Carboxyl, Carbamoyl, Thiocarbamoyl, Cyano, $C_1$-$C_6$-Alkoxycarbonyl, Trifluormethyl, Hydroxy-$C_1$-$C_3$-Alkyl oder $C_1$-$C_4$-Alkanoyl in Betracht kommen.

Wenn in der Formel I substituierte Alkylgruppen auftreten, können als Substituenten z.B. Hydroxy, $C_1$-$C_6$-Alkoxy, Phenyl-$C_1$-$C_4$-alkoxy, Phenoxy, Amino, $C_1$-$C_6$-Mono- oder Dialkylamino, Phenyl-$C_1$-$C_6$-alkylamino, Diphenyl-$C_1$-$C_4$-Alkylamino, Phenylamino, Diphenylamino, $C_3$-$C_6$-Mono- oder Dicycloalkylamino, $C_3$-$C_6$-Cycloalkyl, Hydroxy-$C_1$-$C_6$-alkylamino, Morpholino, Piperazino, N-($C_1$-$C_6$-Alkyl)piperazino, Thiomorpholino, Piperidino, Pyrrolidino, Hexamethylenimino, Thien-2-yl, Furan-2-yl, 1H-Pyrrol-2-yl, Pyridin-2-yl, Pyridin-3-yl, Pyridin-4-yl, Phenyl, Carboxyl, $C_1$-$C_3$-Alkoxy-carbonyl, Carbamoyl, $C_1$-$C_4$-Mono- oder Dialkylcarbamoyl, Cyano, Thiocarbamoyl, phenoxycarbonyl, Phenyl-$C_1$-$C_4$-alkoxycarbonyl, Mono- oder Diphenylcarbamoyl, Mono- oder Bis(phenyl-$C_1$-$C_4$-alkyl)carbamoyl, Mono- oder Diphenylthiocarbamoyl, Mono-oder Bis(phenyl-$C_1$-$C_4$-alkyl)thiocarbamoyl, $C_1$-$C_6$-Alkylthio, Phenylthio oder Phenyl-$C_1$-$C_4$-alkylthio in Betracht kommen.

Wenn in der Formel I substituiertes Alkenyl-, Alkinyl- oder Cycloalkylgruppen auftreten, können als Substituenten z.B. Fluor, Chlor oder Brom in Betracht kommen.

Wenn die Reste $T^2$ und $T^3$ zusammen mit dem sie verbindenden Stickstoffatom einen 5- bis 7-gliedrigen gesättigten heterocyclischen Rest, der gegebenenfalls weitere Heteroatome enthält, bedeuten, so können dafür z.B. Pyrrolidino, Piperidino, Morpholino, Thiomorpholino, Thiomorpholino-S,S-dioxid, Piperazino, N-($C_1$-$c_4$-Alkyl)piperazino oder Hexamethylenimino in Betracht kommen.

Reste $T^1$, $T^2$, $T^3$ und $T^5$ sind beispielsweise Methyl, Ethyl, Propyl, Isopropyl, Butyl, Isobutyl, sec-Butyl, Pentyl, Isopentyl, Neopentyl, tertPentyl oder Hexyl.

Reste $R^4$ sind beispielsweise Formyl, Acetyl, Propionyl, Butyryl, Isobutyryl, Pentanoyl oder Hexanoyl.

(Die im folgenden verwendeten Bezeichnungen Isooctyl, Isononyl, Isodecyl und Isotridecyl sind Trivialbezeichnungen und stammen von den nach der Oxosynthese erhaltenen Alkoholen (vgl. dazu Ullmanns Encyklopädie der technischen Chemie, 4. Auflage, Band 7, Seiten 215 bis 217, sowie Band 11, Seiten 435 und 436.)

Der Rest -NQ leitet sich von primären Aminen der Formel $H_2NQ$ ab. Als solche sind beispielsweise Methylamin, Ethylamin, Propylamin, Isopropylamin, Butylamin, Isobutylamin, sec-Butylamin, tert-Butylamin, Pentylamin, Isopentylamin, Neopentylamin, Hexylamin, Heptylamin, n-Octylamin, Isooctylamin, 2-Ethylhexylamin, Nonylamin, Isononylamin, Decylamin, Isodecylamin, Undecylamin, Dodecylamin, Tridecylamin, Isotridecylamin, Tetradecylamin, Pentadecylamin, Hexadecylamin, Heptadecylamin, Octadecylamin, Nonadecylamin, Eicosylamin, Allylamin, Methallylamin, Propargylamin, Cyclopropylamin, Cyclobutylamin, Cyclopentylamin, Cyclohexylamin, Cycloheptylamin, Cyclooctylamin, Cyclononylamin, Cyclodecylamin, Cycloundecylamin, Cyclododecylamin, 2-Hydroxyethylamin, 2-Methoxyethylamin, 2-Ethoxyethylamin, 3-Hydroxypropylamin, 3-Methoxypropylamin, 3-Ethoxypropylamin, 3-(2-Phenoxyethoxy)propylamin, 3-Benzyloxypropylamin, 2-(N,N-Dimethylamino)ethylamin, 2-(N,N-Diethylamino)ethylamin, 3-(N,N-Dimethylamino)-propylamin, 3-(N,N-Diethylamino)propylamin, Benzylamin, 2-Phenylethylamin, 3-Phenylpropylamin, Anilin, 2-Hydroxyanilin, 3-Hydroxyanilin, 4-Hydroxyanilin, o-Anisidin, m-Anisidin, p-Anisidin, o-Phenetidin, m-Phenetidin, p-Phenetidin, 2-Chloranilin, 3-Chloranilin, 3-Nitroanilin, 4-Nitroanilin, o-Toluidin, m-Toluidin, p-Toluidin, 1,2-Phenylendiamin, 1,3-Phenylendiamin, 1,4-Phenylendiamin, 2-Trifluormethylanilin, 3-Trifluormethylanilin, 4-Trifluormethylanilin, 2-Ethylanilin, 3-Ethylanilin, 4-Ethylanilin, 2-Aminopyridin, 3-Aminopyridin, 4-Aminopyridin, Glycinmethylester, Glycinethylester, Glycinpropylester, Glycinbutylester, Hydrazin, N,N-Dimethylhydrazin oder Phenylhydrazin zu nennen.

Reste $T^4$ sind z.B. Methoxy, Ethoxy, Propoxy, Isopropoxy, Butoxy, Isobutoxy, sec-Butoxy, Pentyloxy, Isopentyloxy, Neopentyloxy oder Hexyloxy.

Der Rest $T^6$ leitet sich z.B. von methylenaktiven Verbindungen der Formel $H_2T^6$ ab. Solche Verbindungen gehorchen beispielsweise der Formel

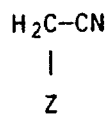 ,

in der Z Cyano, Nitro, $C_1$-$C_6$-Alkanoyl, Benzoyl, $C_1$-$C_4$-Alkylsulfonyl, Phenylsulfonyl, Carboxyl, $C_1$-$C_4$-Alkoxycarbonyl, Carbamoyl oder $C_1$-$C_4$-Mono-oder Dialkylcarbamoyl bedeutet, oder der Formel

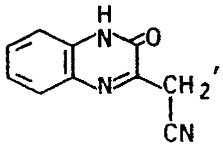, 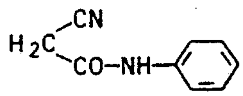, 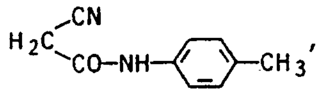, 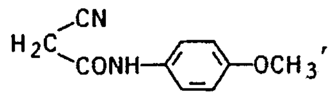

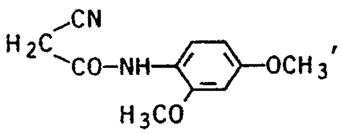, 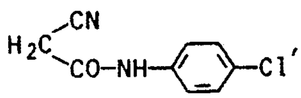, 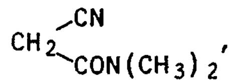, 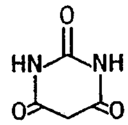,

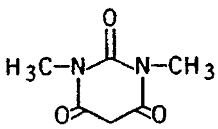, 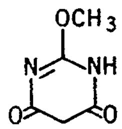, 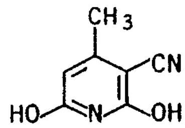, 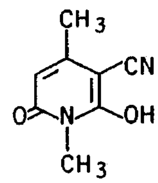, 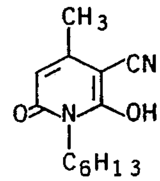,

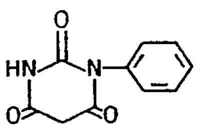, 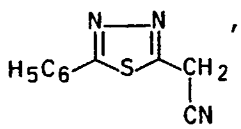, 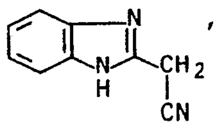, 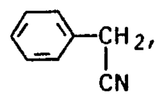, 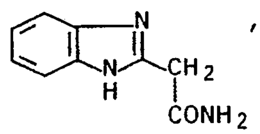,

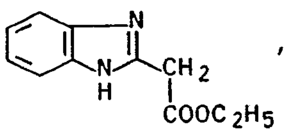, 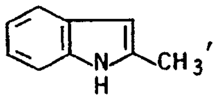, 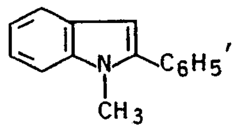, 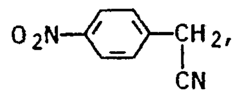

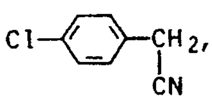, 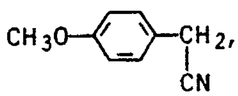, 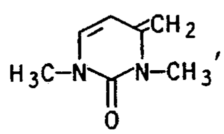, $CH_3-NO_2$, $C_2H_5-NO_2$,

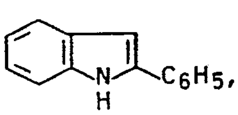, 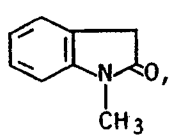, 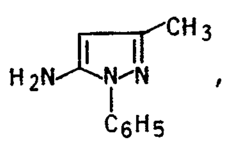, 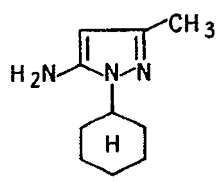, 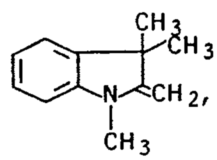

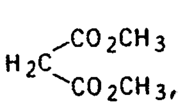, 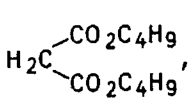, 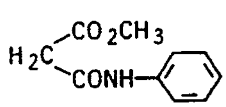 oder 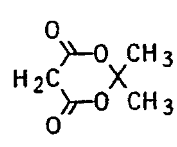.

Einzelne besonders wichtige Verbindungen sind z.B.:

$H_2C(CN)_2$, 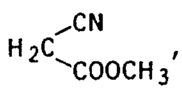, 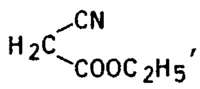, 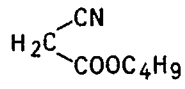, 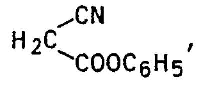, 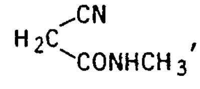,

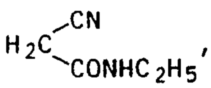, 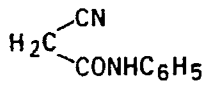, 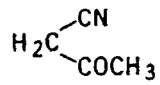, 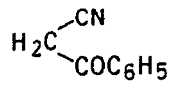, 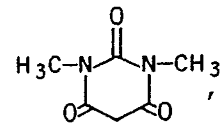,

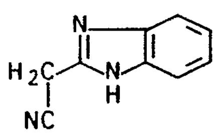, 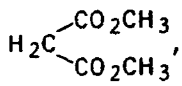, 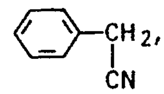, 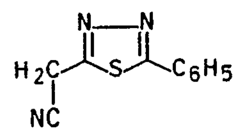 oder 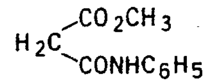.

Reste Y sind beispielsweise Methylamino, Ethylamino, Propylamino, Isopropylamino, Butylamino, Isobutylamino, sec-Butylamino, Pentylamino, Isopentylamino, Neopentylamino, Hexylamino, Heptylamino, Octylamino, Isooctylamino, 2-Ethylhexylamino, Nonylamino, Isononylamino, Decylamino, Isodecylamino, Undecylamino, Dodecylamino, Tridecylamino, Isotridecylamino, Tetradecylamino, Pentadecylamino, Hexadecylamino, Heptadecylamino, Octadecylamino, Nonadecylamino, Eicosylamino, (R)(-)-1-Cyclohexylethylamino, (S)-(+)-1-Cyclohexylethylamino, 2-Cyclohexylethylamino, 2-Hydroxyethylamino, 2-Methoxyethylamino, 2-Ethoxyethylamino, 2-Phenoxyethylamino, 2-(2'-Hydroxyethoxy)ethylamino, 2-(2' -Hydroxyethyl)-aminoethylamino, 2-Aminoethylamino, 2-Dimethylaminoethylamino, 2-Diethylaminoethylamino, 2-Diisopropylaminoethylamino, 2-(Piperazin-1-yl)ethylamino, 3-Hydroxy-n-propylamino, 3-Methoxy-n-propylamino, 3-Ethoxy-n-propylamino, 3-Isopropoxy-n-propylamino, 3-Phenoxy-n-propylamino, 3-Benzyloxy-n-propylamino, 3-(2' -Methoxyethoxy)-n-propylamino, 3-(2' -Ethoxyethoxy)-n-propylamino, 3-(2' -Phenoxyethoxy)-n-propylamino, 3-Amino-n-propylamino, 3-Dimethylamino-n-propylamino, 3-Diethylamino-n-propylamino, 3-Di-n-propylamino-n-propylamino, 3-Diisopropylamino-n-propylamino, 3-Di-n-butylamino-n-propylamino, 3-Morpholin-l-yl-n-propylamino, 4-Hydroxy-n-butylamino, 4-Amino-n-butylamino, 5-Hydroxy-n-pentylamino, 5-Amino-n-pentylamino, 6-Hydroxy-n-hexylamino, 6-Amino-n-hexylamino, 7-Hydroxy-n-heptylamino, 7-Amino-n-heptylamino, 8-Hydroxy-n-octylamino, 8-Amino-n-octylamino, 9-Hydroxy-n-nonylamino, 9-Amino-n-nonylamino, 10-Hydroxy-n-decylamino, 10-Amino-n-decylamino, 11-Amino-n-undecylamino, 4-Diethylamino-1-methyl-n-butylamino, 2-Hydroxy-n-propylamino, 1-Ethyl-2-hydroxyethylamino, 2-Hydroxy-1,1-dimethylethylamino, 1,1-Bis(hydroxymethyl)ethylamino, 1-Ethyl-2-hydroxyethylamino, 2-Hydroxycarbonylethylamino, 3-Hydroxycarbonyl-n-propylamino, 4-Hydroxycarbonyl-n-butylamino, 5-Hydroxycarbonyl-n-pentylamino, 10-Hydroxycarbonyl-n-decylamino, Phenylamino, o-Methylphenylamino, m-Methylphenylamino, p-Methylphenylamino, o-Ethylphenylamino, m-Ethylphenylamino, p-Ethylphenylamino, o-Trifluormethylphenylamino, m-Trifluormethylphenylamino, p-Trifluormethylphenylamino, o-Hydroxyphenylamino, m-Hydroxyphenylamino, p-Hydroxyphenylamino, o-Methoxyphenylamino, m-Methoxyphenylamino, p-Methoxyphenylamino, o-Ethoxyphenylamino, m-Ethoxyphenylamino, p-Ethoxyphenylamino, o-Chlorphenylamino, m-Chlorphenylamino, p-Chlorphenylamino, o-Fluorphenylamino, m-Fluorphenylamino, p-Fluorphenylamino, o-Bromphenylamino, m-Bromphenylamino, p-Bromphenylamino, o-Iodphenylamino, m-Iodphenylamino, p-Iodphenylamino, o-Aminophenylamino, m-Aminophenylamino, p-Aminophenylamino, p-Acetylaminophenylamino, o-Hydroxymethylphenylamino, m-Hydroxymethylphenylamino, p-Hydroxymethylphenylamino, o-Hydroxycarbonylphenylamino, m-Hydroxycarbonylphenylamino, p-Hydroxycarbonylphenylamino, o-Aminocarbonylphenylamino, m-Aminocarbonylphenylamino, p-Aminocarbonylphenylamino, o-Cyanophenylamino, m-Cyanophenylamino, p-Cyanophenylamino, 2,6-Dimethylphenylamino, 3,5-Dimethylphenylamino, 2-Hydroxy-5-methylphenylamino, 2-Hydroxy-4-methylphenylamino, 4-Hydroxy-2-methylphenylamino, 2-Hydroxy-5-chlorphenylamino, Imidazol-1-yl, Pyrazol-1-yl, Pyrid-2-ylamino, Pyrid-3-ylamino, Pyrid-4-ylamino, Pyrid-3-ylmethylamino, Fur-2-ylmethylamino, Thienylamino, Benzylamino, o-Methoxybenzylamino, p-Methoxybenzylamino, p-Fluorbenzylamino, o-Chlorbenzylamino, p-Chlorbenzylamino, 3,4-Dimethoxybenzylamino, 2-Phenylethylamino, (+/-)-α-Methylbenzylamino, (+)-α-Methylbenzylamino, (-)-α-Methylbenzylamino, 3-Phenyl-n-propylamino, 1-Methyl-3-phenyl-n-propylamino, Cyclopropylamino, Cyclobutylamino, Cyclopentylamino, Cyclohexylamino, Cycloheptylamino, Cyclooctylamino, Cyclononylamino, Cyclodecylamino, Cyclododecylamino, Allylamino, Propargylamino, Methallylamino, Dimethylamino, Diethylamino, Di-n-propylamino, Di-n-butylamino, Diisopropylamino, Diisobutylamino, Di-sec-butylamino, Di-n-pentylamino, Diisopentylamino, Di-n-hexylamino, Di(2-ethylhexyl)-

amino, Di-n-octylamino, Diallylamino, Dicyclohexylamino, N-Methyl-n-butylamino, N-Methylcyclohexylamino, N-Ethylcyclohexylamino, N-Methylethanolamino, N-Ethyl-1,3-dimethyl-n-propylamino, N-Ethylethanolamino, N-Ethyl-2-hydroxy-n-propylamino, N-tert-Butylethanolamino, N-Methylphenylamino, N-Ethylphenylamino, N-n-Propylphenylamino, N-Isopropylphenylamino, N-n-Butylphenylamino, Dibenzylamino, N-Methylbenzylamino, N-Ethylbenzylamino, N-Isopropylbenzylamino, N-tert-Butylbenzylamino, N-(2-Hydroxyethyl)benzylamino, N-Phenylbenzylamino, N-Benzyl-2-phenylethylamino, Pyrrolidino, Piperidino, Hexamethylenimino, Morpholino, N-Methylpiperazino, Piperazino, N-Ethylpiperazino oder Thiomorpholino.

Reste $R^3$ sind z.B. Mercapto, Methylthio, Ethylthio, Propylthio, Isopropylthio, Butylthio, Isobutylthio, sec-Butylthio, Pentylthio, Isopentylthio, Neopentylthio, tert-Pentylthio, Hexylthio, Heptylthio, Octylthio, 2-Ethylhexylthio, Benzylthio, 1- oder 2-Phenylethylthio, Cyclopentylthio, Cyclohexylthio, Cycloheptylthio, Allylthio, Methallylthio, Propargylthio, Phenylthio, Methylsulfinyl, Ethylsulfinyl, Propylsulfinyl, Isopropylsulfinyl, Butylsulfinyl, Isobutylsulfinyl, sec-Butylsulfinyl Pentylsulfinyl, Isopentylsulfinyl, Neopentylsulfinyl, tert-Pentylsulfinyl, Hexylsulfinyl, Heptylsulfinyl, Octylsulfinyl, 2-Ethylhexylsulfinyl, Benzylsulfinyl, 1- oder 2-Phenylethylsulfinyl, Cyclopentylsulfinyl, Cyclohexylsulfinyl, Cycloheptylsulfinyl, Allylsulfinyl, Methallylsulfinyl, Propargylsulfinyl, Phenylsulfinyl, Methylsulfonyl, Ethylsulfonyl, Propylsulfony, Isopropylsulfonyl Butylsulfonyl, Isobutylsulfonyl, sec-Butylsulfonyl, Pentylsulfonyl, Isopentylsulfonyl Neopentylsulfony, tert-Pentylsulfonyl, Hexylsulfonyl, Heptylsulfonyl, Octylsulfonyl, 2-Ethylhexylsulfonyl, Benzylsulfonyl, 1- oder 2-Phenylethylsulfonyl, Cyclopentylsulfonyl, Cycloheylsulfonyl, Cycloheptylsulfonyl, Allylsulfonyl, Methallylsulfonyl, Propargylsulfonyl oder Phenysulfonyl.

Bevorzugt sind Aminothiazole der Formel I, in der
$R^1$ und $R^2$ jeweils Wasserstoff oder zusammen den Rest

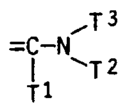

bedeuten, worin $T^1$ für Wasserstoff, $C_1$-$C_4$-Alkyl oder Phenyl und $T^2$ und $T^3$ unabhängig voneinander für $C_1$-$C_4$-Alkyl oder Phenyl, oder zusammen mit dem sie verbindenden Stickstoffatom für Pyrrolidino, Piperidino, Morpholino, Thiomorpholino, Piperazino, N-($C_1$-$C_4$-Alkyl)piperazino oder Hexamethylenimino stehen, und $R^3$ und $R^4$ jeweils die obengenannte Bedeutung besitzen.

Besonders bevorzugt sind Aminothiazole der Formel I, in der $R^1$ und $R^2$ jeweils Wasserstoff oder zusammen den Rest

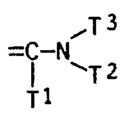

bedeuten, worin $T^1$ für Wasserstoff und $T^2$ und $T^3$ unabhängig voneinander jedoch für $C_1$-$C_4$-Alkyl stehen.

Weiterhin besonders bevorzugt sind Aminothiazole der Formel I, in der $R^4$ Formyl, Acetyl, Propionyl, Butyryl, Benzoyl, Cyano oder einen Rest der Formel

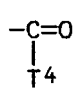

oder

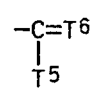

bedeutet, worin $T^5$ für Wasserstoff, Methyl, Ethyl oder Phenyl steht und $T^4$ und $T^6$ jeweils die obengenannte Bedeutung besitzen.

Weiterhin besonders bevorzugt sind Aminothiazole der Formel I, in der Y $C_1$-$C_6$-Mono- oder Dialkylamino, Allylamino, Methallylamino, Propargylamino, $C_2$-$C_6$-Alkylamino, das durch Phenyl, Hydroxy, Phenoxy, Amino oder $C_1$-$C_4$-Mono-oder Dialkylamino substituiert oder durch ein Sauerstoffatom unterbrochen ist, Phenylamino, Pyrrolidino, Piperidino, Morpholino, Piperazino oder N-($C_1$-$C_4$-Alkyl)piperazino bedeutet.

Weiterhin besonders bevorzugt sind Aminothiazole der Formel I, in der $R^3$ $C_1$-$C_6$-Alkylthio, Benzylthio,

Cyclohexylthio, $C_3$-$C_4$-Alkenylthio, Propargylthio, Phenylthio, $C_1$-$C_6$-Alkylsulfonyl, Benzylsulfonyl, Cyclohexylsulfonyl, $C_3$-$C_4$-Alkenylsulfonyl, Propargylsulfonyl oder Phenylsulfonyl bedeutet.

Weiterhin besonders bevorzugt sind Aminothiazole der Formel I, in der $R^4$ Cyano, Formyl oder den Rest

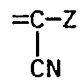

bedeutet, worin Z für Cyano, Nitro oder $C_1$-$C_4$-Alkoxycarbonyl steht.

Insbesondere von Bedeutung sind Aminothiazole der Formel I, in der $R^1$ und $R^2$ jeweils Wasserstoff oder zusammen den Rest $=CH\text{-}N(CH_3)_2$ bedeuten.

Weiterhin insbesondere zu nennen sind Aminothiazole der Formel I, in der $R^4$ Formyl, Cyano oder ein Rest der Formel

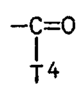

oder

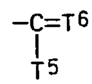

bedeutet, worin $T^4$ für Hydroxy, Methoxy, Ethoxy oder Amino und $T^5$ für Wasserstoff, Methyl, Ethyl oder Phenyl stehen und $T^6$ die obengenannte Bedeutung besitzt.

Hervorzuheben sind Aminothiazhole der Formel I in der
$R^4$ Formyl, Cyano oder ein Rest der Formel

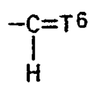

bedeutet, worin $T^6$ die obengenannte Bedeutung besitzt.

Ganz besonders hervorzuheben sind Aminothiazole der Formel Ia

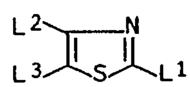

(Ia)

oder deren Tautomere, wobei

$L^1$ Amino,

$L^2$ $C_1$-$C_6$-Alkylthio, Benzylthio, Cyclohexylthio, $C_3$-$C_4$-Alkenylthio, Propargylthio, Phenylthio, $C_1$-$C_6$-Alkylsulfonyl, Benzylsulfonyl, Cyclohexylsulfonyl, $C_3$-$C_4$-Alkenylsulfonyl, Propargylsulfonyl, Phenylsulfonyl, $C_1$-$C_6$-Mono- oder Dialkylamino, Allylamino, Methallylamino, Propargylamino, $C_2$-$C_6$-Alkylamino, das durch Phenyl, Hydroxy, Phenoxy, Amino oder $C_1$-$C_4$-Mono- oder Dialkylamino substituiert oder durch ein Sauerstoffatom unterbrochen ist, Phenylamino, Pyrrolidino, Piperidino, Morpholino, Piperazino oder N-($C_1$-$C_4$-Alkyl)piperazino und

$L^3$ Cyano, Formyl oder den Rest

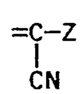

bedeuten, worin Z für Cyano, Nitro oder $C_1$-$C_4$-Alkoxycarbonyl steht.

Von besonderer technischer Bedeutung sind Aminothiazole der Formel I, in der n 0 oder 2 und X Benzy oder Phenyl bedeutet.

Die erfindungsgemäßen Aminothiazole der Formel I können z.B. durch Umsetzung von Halogenaminot-

hiazolen der Formel II

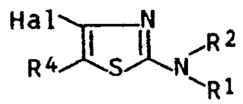

(II),

in der $R^1$, $R^2$ und $R^4$ jeweils die obengenannte Bedeutung besitzen und Hal für Chlor oder Brom steht, mit einer Verbindung der Formel III

$R^3$-H (III),

in der $R^3$ die obengenannte Bedeutung besitzt, erhalten werden. Wenn $R^4$ für den Rest -S(O)$_n$-X steht, verwendet man die Verbindungen dabei in der Regel in Form ihrer Alkalisalze.

Dazu setzt man z.B. das Halogenaminothiazol II mit der Verbindung III (oder deren Alkalisalz) in einem inerten Lösungsmittel (z.B. N,N-Dimethylformamid, N,N-Dimethylacetamid, N-Methylpyrrolidinon, Hexamethylphosphorsäuretrisamid, 1,3-Dimethylimidazolidin-2-on, 1,3-Dimethylhexahydropyrimidin-2-on, 1,2-Diethoxyethan, Benzol, Toluol, Xylol, Methylenchlorid, Chloroform, Methanol, Ethanol, Propanol, Isopropanol, Butanol oder Isobutanol) bei einer Temperatur von -10 bis +150 °C, gegebenenfalls in Gegenwart einer Hilfsbase (z.B. Triethylamin, Tripropylamin, Tributylamin, Pyridin, 4-Dimethylaminopyridin, Natriummethylat, Natriumethylat, Kaliumtert-butylat, Natriumhydrogencarbonat, Natriumcarbonat, Kaliumhydrogencarbonat, Kaliumcarbonat, Natriumhydrid oder Kaliumhydrid) um. Das Molverhältnis II:III beträgt dabei in der Regel 1:2 bis 1:10. Wenn man in Gegenwart einer Hilfsbase arbeitet, so beträgt das Molverhältnis Hilfsbase : III im allgemeinen 1:1 bis 10:1.

In Gegenwart einer Hilfsbase arbeitet man zweckmäßig dann, wenn man Verbindungen der Formel III umsetzt, in der $R^3$ für den Rest -S(O)$_n$-X steht.

Die Halogenaminothiazole der Formel II sind an sich bekannt und z.B. in der US-A-4 395 544 beschrieben oder können analog den dort aufgeführten Methoden erhalten werden.

Die neuen Aminothiazole sind wertvolle Zwischenprodukte für die Synthese von Farbstoffen, Pflanzenschutzmitteln oder Pharmazeutika. Insbesondere dienen sie als Diazokomponente ($R^1$, $R^2$ = H) oder deren Vorstufe für die Herstellung von Azofarbstoffen.

Die folgenden Beispiele sollen die Erfindung näher erläutern.

Beispiel 1

3,7 g Natriumethanolat wurden in 40 ml Ethanol gelöst. Hierzu wurden 4,5 g n-Butanthiol zugegeben und das Gemisch 15 Minuten bei Raumtemperatur gerührt. Anschließend wurden 8,1 g 2-Amino-4-chlor-5-formylthiazol zugegeben und das Gemisch 2 Stunden bei Raumtemperatur gerührt. Danach wurde das Lösungsmittel unter vermindertem Druck abdestilliert und der Rückstand mit 150 ml heißem Wasser verrührt. Die Suspension wurde mit konz. Salzsäure auf einen pH-Wert von 3 eingestellt, auf 10 °C abgekühlt und weitere 2 Stunden bei dieser Temperatur gerührt. Der Niederschlag wurde abgesaugt, mit Wasser nachgewaschen und bei 50 °C im Vakuumtrockenschrank getrocknet. Zur Reinigung wurde aus einem Gemisch aus Wasser und Essigsäure unter Zusatz von Aktivkohle umkristallisiert. Man erhielt 8,1 g (75 % d. Th.) der Verbindung der Formel

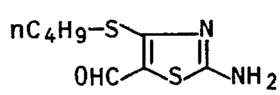

vom Schmelzpunkt 131 bis 132 °C. Die IR-, NMR-, UV- und Massenspektren sowie die Elementaranalyse stimmen mit oben angegebener Konstitutionsformel überein.

Beispiel 2

16,25 g 2-Amino-4-chlor-5-formylthiazol wurden in 100 ml N,N-Dimethylformamid gelöst. Hierzu wurden

19,6 g Natrium-benzolsulfinat gegeben. Das Reaktionsgemisch wird eine Stunde bei Raumtemperatur und 4 Stunden bei 70°C gerührt. Anschließend wurde das Gemisch auf 600 ml Eiswasser gegeben und der anfallende Niederschlag abgesaugt, nachgewaschen mit Wasser und unter vermindertem bei 50°C getrocknet. Man erhielt 21,6 g (81 % d. Th.) der Verbindung

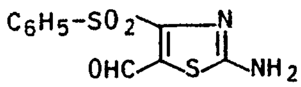

vom Schmelzpunkt 166 bis 167°C. Die IR-, NMR-, UV- und Massenspektren sowie die Elementaranalyse stimmen mit oben angegebener Formel überein.

In analoger Weise lassen sich die in der folgenden Tabelle 1 aufgeführten Verbindungen der Formel

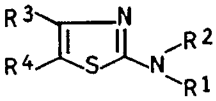

herstellen.

Tabelle 1

| Bsp. Nr. | $R^3$ | $NR^1R^2$ | $R^4$ | Schmelzpunkt (°C) |
|---|---|---|---|---|
| 3 | SH | $NH_2$ | CHO | |
| 4 | $S-CH_3$ | $NH_2$ | CHO | |
| 5 | $S-C_2H_5$ | $NH_2$ | CHO | |
| 6 | $S-nC_3H_7$ | $NH_2$ | CHO | 124 - 124,5 |
| 7 | $S-isoC_3H_7$ | $NH_2$ | CHO | |
| 8 | $S-isoC_4H_9$ | $NH_2$ | CHO | |
| 9 | $S-secC_4H_9$ | $NH_2$ | CHO | |
| 10 | $S-tertC_4H_9$ | $NH_2$ | CHO | |
| 11 | $S-nC_5H_{11}$ | $NH_2$ | CHO | |
| 12 | $S-neoC_5H_{11}$ | $NH_2$ | CHO | |
| 13 | $S-nC_6H_{13}$ | $NH_2$ | CHO | 123 - 125 |
| 14 | $S-nC_7H_{15}$ | $NH_2$ | CHO | |
| 15 | $S-nC_8H_{17}$ | $NH_2$ | CHO | 106 - 107 |
| 16 | $S-nC_9H_{19}$ | $NH_2$ | CHO | |
| 17 | $S-nC_{10}H_{21}$ | $NH_2$ | CHO | |
| 18 | $S-nC_{11}H_{23}$ | $NH_2$ | CHO | |
| 19 | $S-nC_{12}H_{25}$ | $NH_2$ | CHO | |
| 20 | $S-nC_{13}H_{27}$ | $NH_2$ | CHO | |
| 21 | $S-nC_{14}H_{29}$ | $NH_2$ | CHO | |
| 22 | $S-nC_{15}H_{31}$ | $NH_2$ | CHO | |
| 23 | $S-nC_{16}H_{33}$ | $NH_2$ | CHO | |
| 24 | $S-nC_{17}H_{35}$ | $NH_2$ | CHO | |
| 25 | $S-nC_{18}H_{37}$ | $NH_2$ | CHO | |
| 26 | $S-nc_{19}H_{39}$ | $NH_2$ | CHO | |
| 27 | $S-nC_{20}H_{41}$ | $NH_2$ | CHO | |
| 28 | S-Cyclopropyl | $NH_2$ | CHO | |
| 29 | S-Cyclopentyl | $NH_2$ | CHO | |
| 30 | S-Cyclohexyl | $NH_2$ | CHO | 162 - 163,5 |
| 31 | S-Cycloheptyl | $NH_2$ | CHO | |
| 32 | S-Cyclooctyl | $NH_2$ | CHO | |
| 33 | $S-C_6H_5$ | $NH_2$ | CHO | 174 - 175 |
| 34 | $S-CH_2C_6H_5$ | $NH_2$ | CHO | 153 - 155 |
| 35 | $S-(CH_2)_2-C_6H_5$ | $NH_2$ | CHO | |
| 36 | $S-CH_2CH_2OH$ | $NH_2$ | CHO | |
| 37 | $S-CH_2-CH=CH_2$ | $NH_2$ | CHO | |
| 38 | $S-CH_2-C\equiv CH$ | $NH_2$ | CHO | |
| 39 | SH | $NH_2$ | CN | |

| Bsp. Nr. | $R^3$ | $NR^1R^2$ | $R^4$ | Schmelzpunkt (°C) |
|---|---|---|---|---|
| 40 | $S-C_2H_5$ | $NH_2$ | CN | |
| 41 | $S-nC_3H_7$ | $NH_2$ | CN | |
| 42 | $S-isoC_3H_7$ | $NH_2$ | CN | |
| 43 | $S-nC_4H_9$ | $NH_2$ | CN | |
| 44 | $S-isoC_4H_9$ | $NH_2$ | CN | |
| 45 | $S-secC_4H_9$ | $NH_2$ | CN | |
| 46 | $S-tertC_4H_9$ | $NH_2$ | CN | |
| 47 | $S-nC_5H_{11}$ | $NH_2$ | CN | |
| 48 | $S-neoC_5H_{11}$ | $NH_2$ | CN | |
| 49 | $S-nC_6H_{13}$ | $NH_2$ | CN | |
| 50 | $S-nC_7H_{15}$ | $NH_2$ | CN | |
| 51 | $S-nC_8H_{17}$ | $NH_2$ | CN | |
| 52 | $S-nC_9H_{19}$ | $NH_2$ | CN | |
| 53 | $S-nC_{10}H_{21}$ | $NH_2$ | CN | |
| 54 | $S-nC_{11}H_{23}$ | $NH_2$ | CN | |
| 55 | $S-nC_{12}H_{23}$ | $NH_2$ | CN | |
| 56 | $S-nC_{13}H_{25}$ | $NH_2$ | CN | |
| 57 | $S-nC_{14}H_{29}$ | $NH_2$ | CN | |
| 58 | $S-nC_{15}H_{31}$ | $NH_2$ | CN | |
| 59 | $S-nC_{16}H_{33}$ | $NH_2$ | CN | |
| 60 | $S-nC_{17}H_{35}$ | $NH_2$ | CN | |
| 61 | $S-nC_{18}H_{37}$ | $NH_2$ | CN | |
| 62 | $S-nC_{19}H_{39}$ | $NH_2$ | CN | |
| 63 | $S-nC_{20}H_{41}$ | $NH_2$ | CN | |
| 64 | S-Cyclopropyl | $NH_2$ | CN | |
| 65 | S-Cyclopentyl | $NH_2$ | CN | |
| 66 | S-Cyclohexyl | $NH_2$ | CN | |
| 67 | S-Cycloheptyl | $NH_2$ | CN | |
| 68 | S-Cyclooctyl | $NH_2$ | CN | |
| 69 | $S-C_6H_5$ | $NH_2$ | CN | |
| 70 | $S-CH_2C_6H_5$ | $NH_2$ | CN | |
| 71 | $S-(CH_2)_2-C_6H_5$ | $NH_2$ | CN | |
| 72 | $S-CH_2CH_2OH$ | $NH_2$ | CN | |
| 73 | $S-CH_2-CH=CH_2$ | $NH_2$ | CN | |
| 74 | $S-CH_2-C\equiv CH$ | $NH_2$ | CN | |
| 75 | $S-CH_2-C(CH_3)=CH_2$ | $NH_2$ | CHO | |
| 76 | $SO_2-CH_3$ | $NH_2$ | CHO | |
| 77 | $SO_2-C_2H_5$ | $NH_2$ | CHO | |
| 78 | $SO_2-nC_3H_7$ | $NH_2$ | CHO | |

| Bsp. Nr. | $R^3$ | $NR^1R^2$ | $R^4$ | Schmelzpunkt (°C) |
|---|---|---|---|---|
| 79 | $SO_2-isoC_3H_7$ | $NH_2$ | CHO | |
| 80 | $SO_2-nC_4H_9$ | $NH_2$ | CHO | |
| 81 | $SO_2-isoC_4H_9$ | $NH_2$ | CHO | |
| 82 | $SO_2-secC_4H_9$ | $NH_2$ | CHO | |
| 83 | $SO_2-tertC_4H_9$ | $NH_2$ | CHO | |
| 84 | $SO_2-nC_5H_{11}$ | $NH_2$ | CHO | |
| 85 | $SO_2-neoC_5H_{11}$ | $NH_2$ | CHO | |
| 86 | $SO_2-nC_6H_{13}$ | $NH_2$ | CHO | |
| 87 | $SO_2-nC_7H_{15}$ | $NH_2$ | CHO | |
| 88 | $SO_2-nC_8H_{17}$ | $NH_2$ | CHO | |
| 89 | $SO_2-nC_9H_{19}$ | $NH_2$ | CHO | |
| 90 | $SO_2-nC_{10}H_{21}$ | $NH_2$ | CHO | |
| 91 | $SO_2-nC_{11}H_{23}$ | $NH_2$ | CHO | |
| 92 | $SO_2-nC_{12}H_{25}$ | $NH_2$ | CHO | |
| 93 | $SO_2-nC_{13}H_{27}$ | $NH_2$ | CHO | |
| 94 | $SO_2-nC_{14}H_{29}$ | $NH_2$ | CHO | |
| 95 | $SO_2-nC_{15}H_{31}$ | $NH_2$ | CHO | |
| 96 | $SO_2-nC_{16}H_{33}$ | $NH_2$ | CHO | |
| 97 | $SO_2-nC_{17}H_{35}$ | $NH_2$ | CHO | |
| 98 | $SO_2-nC_{18}H_{37}$ | $NH_2$ | CHO | |
| 99 | $SO_2-nC_{19}H_{39}$ | $NH_2$ | CHO | |
| 100 | $SO_2-nC_{20}H_{41}$ | $NH_2$ | CHO | |
| 101 | $SO_2$-Cyclopropyl | $NH_2$ | CHO | |
| 102 | $SO_2$-Cyclopentyl | $NH_2$ | CHO | |
| 103 | $SO_2$-Cyclohexyl | $NH_2$ | CHO | |
| 104 | $SO_2$-Cycloheptyl | $NH_2$ | CHO | |
| 105 | $SO_2$-Cyclooctyl | $NH_2$ | CHO | |
| 106 | $SO_2-C_6H_5$ | $NH_2$ | $CH=C(CN)\ (COOC_2H_5)$ | |
| 107 | $SO_2-CH_2C_6H_5$ | $NH_2$ | CHO | |
| 108 | $SO_2-(CH_2)_2-C_6H_5$ | $NH_2$ | CHO | |
| 109 | $SO_2-CH_2CH_2OH$ | $NH_2$ | CHO | |
| 110 | $SO_2-CH_2-CH=CH_2$ | $NH_2$ | CHO | |
| 111 | $SO_2-CH_2-C\equiv CH$ | $NH_2$ | CHO | |
| 112 | $S-CH_2-C(CH_3)=CH_2$ | $NH_2$ | CN | |
| 113 | $SO_2-CH_3$ | $NH_2$ | CN | |
| 114 | $SO_2-C_2H_5$ | $NH_2$ | CN | |
| 115 | $SO_2-nC_3H_7$ | $NH_2$ | CN | |
| 116 | $SO_2-isoC_3H_7$ | $NH_2$ | CN | |
| 117 | $SO_2-nC_4H_9$ | $NH_2$ | CN | |

| Bsp. Nr. | $R^3$ | $NR^1R^2$ | $R^4$ | Schmelzpunkt (°C) |
|---|---|---|---|---|
| 118 | $SO_2$-iso$C_4H_9$ | $NH_2$ | CN | |
| 119 | $SO_2$-sec$C_4H_9$ | $NH_2$ | CN | |
| 120 | $SO_2$-tert$C_4H_9$ | $NH_2$ | CN | |
| 121 | $SO_2$-n$C_5H_{11}$ | $NH_2$ | CN | |
| 122 | $SO_2$-neo$C_5H_{11}$ | $NH_2$ | CN | |
| 123 | $SO_2$-n$C_6H_{13}$ | $NH_2$ | CN | |
| 124 | $SO_2$-n$C_7H_{15}$ | $NH_2$ | CN | |
| 125 | $SO_2$-n$C_8H_{17}$ | $NH_2$ | CN | |
| 126 | $SO_2$-n$C_9H_{19}$ | $NH_2$ | CN | |
| 127 | $SO_2$-n$C_{10}H_{21}$ | $NH_2$ | CN | |
| 128 | $SO_2$-n$C_{11}H_{23}$ | $NH_2$ | CN | |
| 129 | $SO_2$-n$C_{12}H_{25}$ | $NH_2$ | CN | |
| 130 | $SO_2$-n$C_{13}H_{27}$ | $NH_2$ | CN | |
| 131 | $SO_2$-n$C_{14}H_{29}$ | $NH_2$ | CN | |
| 132 | $SO_2$-n$C_{15}H_{31}$ | $NH_2$ | CN | |
| 133 | $SO_2$-n$C_{16}H_{33}$ | $NH_2$ | CN | |
| 134 | $SO_2$-n$C_{17}H_{35}$ | $NH_2$ | CN | |
| 135 | $SO_2$-n$C_{18}H_{37}$ | $NH_2$ | CN | |
| 136 | $SO_2$-n$C_{19}H_{39}$ | $NH_2$ | CN | |
| 137 | $SO_2$-n$C_{20}H_{41}$ | $NH_2$ | CN | |
| 138 | $SO_2$-Cyclopropyl | $NH_2$ | CN | |
| 139 | $SO_2$-Cyclopentyl | $NH_2$ | CN | |
| 140 | $SO_2$-Cyclohexyl | $NH_2$ | CN | |
| 141 | $SO_2$-Cycloheptyl | $NH_2$ | CN | |
| 142 | $SO_2$-Cyclooctyl | $NH_2$ | CN | |
| 143 | $SO_2-C_6H_5$ | $NH_2$ | CN | |
| 144 | $SO_2-CH_2C_6H_5$ | $NH_2$ | CN | |
| 145 | $SO_2-(CH_2)_2-C_6H_5$ | $NH_2$ | CN | |
| 146 | $SO_2-CH_2CH_2OH$ | $NH_2$ | CN | |
| 147 | $SO_2-CH_2-CH{=}CH_2$ | $NH_2$ | CN | |
| 148 | $SO_2-CH_2-C{\equiv}CH$ | $NH_2$ | CN | |
| 149 | SH | $NH_2$ | COOH | |
| 150 | $S-CH_3$ | $NH_2$ | COOH | |
| 151 | $S-C_2H_5$ | $NH_2$ | COOH | |
| 152 | S-n$C_3H_7$ | $NH_2$ | COOH | |
| 153 | S-iso$C_3H_7$ | $NH_2$ | COOH | |
| 154 | S-iso$C_4H_9$ | $NH_2$ | COOH | |
| 155 | S-sec$C_4H_9$ | $NH_2$ | COOH | |
| 156 | S-tert$C_4H_9$ | $NH_2$ | COOH | |

| Bsp. Nr. | $R^3$ | $NR^1R^2$ | $R^4$ | Schmelzpunkt (°C) |
|---|---|---|---|---|
| 157 | $S-nC_5H_{11}$ | $NH_2$ | COOH | |
| 158 | $S-neoC_5H_{11}$ | $NH_2$ | COOH | |
| 159 | $S-nC_6H_{13}$ | $NH_2$ | COOH | |
| 160 | $S-nC_7H_{15}$ | $NH_2$ | COOH | |
| 161 | $S-nC_8H_{17}$ | $NH_2$ | COOH | |
| 162 | $S-nC_9H_{19}$ | $NH_2$ | COOH | |
| 163 | $S-nC_{10}H_{21}$ | $NH_2$ | COOH | |
| 164 | $S-nC_{11}H_{23}$ | $NH_2$ | COOH | |
| 165 | $S-nC_{12}H_{25}$ | $NH_2$ | COOH | |
| 166 | $S-nC_{13}H_{27}$ | $NH_2$ | COOH | |
| 167 | $S-nC_{14}H_{29}$ | $NH_2$ | COOH | |
| 168 | $S-nC_{15}H_{31}$ | $NH_2$ | COOH | |
| 169 | $S-nC_{16}H_{33}$ | $NH_2$ | COOH | |
| 170 | $S-nC_{17}H_{35}$ | $NH_2$ | COOH | |
| 171 | $S-nC_{18}H_{37}$ | $NH_2$ | COOH | |
| 172 | $S-nC_{20}H_{39}$ | $NH_2$ | COOH | |
| 173 | $S-nC_{20}H_{41}$ | $NH_2$ | COOH | |
| 174 | S-Cyclopropyl | $NH_2$ | COOH | |
| 175 | S-Cyclopentyl | $NH_2$ | COOH | |
| 176 | S-Cyclohexyl | $NH_2$ | COOH | |
| 177 | S-Cycloheptyl | $NH_2$ | COOH | |
| 178 | S-Cyclooctyl | $NH_2$ | COOH | |
| 179 | $S-C_6H_5$ | $NH_2$ | COOH | |
| 180 | $S-CH_2C_6H_5$ | $NH_2$ | COOH | |
| 181 | $S-(CH_2)_2-C_6H_5$ | $NH_2$ | COOH | |
| 182 | $S-CH_2CH_2OH$ | $NH_2$ | COOH | |
| 183 | $S-CH_2-CH=CH_2$ | $NH_2$ | COOH | |
| 184 | $S-CH_2-C\equiv CH$ | $NH_2$ | COOH | |
| 185 | SH | $NH_2$ | $COOCH_3$ | |
| 186 | $S-CH_3$ | $NH_2$ | $COOCH_3$ | |
| 187 | $S-C_2H_5$ | $NH_2$ | $COOCH_3$ | |
| 188 | $S-nC_3H_7$ | $NH_2$ | $COOCH_3$ | |
| 189 | $S-isoC_3H_7$ | $NH_2$ | $COOCH_3$ | |
| 190 | $S-nC_4H_9$ | $NH_2$ | $COOCH_3$ | |
| 191 | $S-isoC_4H_9$ | $NH_2$ | $COOCH_3$ | |
| 192 | $S-secC_4H_9$ | $NH_2$ | $COOCH_3$ | |
| 193 | $S-tertC_4H_9$ | $NH_2$ | $COOCH_3$ | |
| 194 | $S-nC_5H_{11}$ | $NH_2$ | $COOCH_3$ | |
| 195 | $S-neoC_5H_{11}$ | $NH_2$ | $COOCH_3$ | |

| Bsp. Nr. | $R^3$ | $NR^1R^2$ | $R^4$ | Schmelzpunkt (°C) |
|---|---|---|---|---|
| 196 | $S-nC_6H_{13}$ | $NH_2$ | $COOCH_3$ | |
| 197 | $S-nC_7H_{15}$ | $NH_2$ | $COOCH_3$ | |
| 198 | $S-nC_8H_{17}$ | $NH_2$ | $COOCH_3$ | |
| 199 | $S-nC_9H_{19}$ | $NH_2$ | $COOCH_3$ | |
| 200 | $S-nC_{10}H_{21}$ | $NH_2$ | $COOCH_3$ | |
| 201 | $S-nC_{11}H_{23}$ | $NH_2$ | $COOCH_3$ | |
| 202 | $S-nC_{12}H_{25}$ | $NH_2$ | $COOCH_3$ | |
| 203 | $S-nC_{13}H_{27}$ | $NH_2$ | $COOCH_3$ | |
| 204 | $S-nC_{14}H_{29}$ | $NH_2$ | $COOCH_3$ | |
| 205 | $S-nC_{15}H_{31}$ | $NH_2$ | $COOCH_3$ | |
| 206 | $S-nC_{16}H_{33}$ | $NH_2$ | $COOCH_3$ | |
| 207 | $S-nC_{17}H_{35}$ | $NH_2$ | $COOCH_3$ | |
| 208 | $S-nC_{18}H_{37}$ | $NH_2$ | $COOCH_3$ | |
| 209 | $S-nC_{19}H_{39}$ | $NH_2$ | $COOCH_3$ | |
| 210 | $S-nC_{20}H_{41}$ | $NH_2$ | $COOCH_3$ | |
| 211 | S-Cyclopropyl | $NH_2$ | $COOCH_3$ | |
| 212 | S-Cyclopentyl | $NH_2$ | $COOCH_3$ | |
| 213 | S-Cyclohexyl | $NH_2$ | $COOCH_3$ | |
| 214 | S-Cycloheptyl | $NH_2$ | $COOCH_3$ | |
| 215 | S-Cyclooctyl | $NH_2$ | $COOCH_3$ | |
| 216 | $S-C_6H_5$ | $NH_2$ | $COOCH_3$ | |
| 217 | $S-CH_2C_6H_5$ | $NH_2$ | $COOCH_3$ | |
| 218 | $S-(CH_2)_2-C_6H_5$ | $NH_2$ | $COOCH_3$ | |
| 219 | $S-CH_2CH_2OH$ | $NH_2$ | $COOCH_3$ | |
| 220 | $S-CH_2-CH=CH_2$ | $NH_2$ | $COOCH_3$ | |
| 221 | $S-CH_2-C\equiv CH$ | $NH_2$ | $COOCH_3$ | |
| 222 | $S-CH_2-C(CH_3)=CH_2$ | $NH_2$ | COOH | |
| 223 | $SO_2-CH_3$ | $NH_2$ | COOH | |
| 224 | $SO_2-C_2H_5$ | $NH_2$ | COOH | |
| 225 | $SO_2-nC_3H_7$ | $NH_2$ | COOH | |
| 226 | $SO_2-isoC_3H_7$ | $NH_2$ | COOH | |
| 227 | $SO_2-nC_4H_9$ | $NH_2$ | COOH | |
| 228 | $SO_2-isoC_4H_9$ | $NH_2$ | COOH | |
| 229 | $SO_2-secC_4H_9$ | $NH_2$ | COOH | |
| 230 | $SO_2-tertC_4H_9$ | $NH_2$ | COOH | |
| 231 | $SO_2-nC_5H_{11}$ | $NH_2$ | COOH | |
| 232 | $SO_2-neoC_5H_{11}$ | $NH_2$ | COOH | |
| 233 | $SO_2-nC_6H_{13}$ | $NH_2$ | COOH | |
| 234 | $SO_2-nC_7H_{15}$ | $NH_2$ | COOH | |

| Bsp. Nr. | $R^3$ | $NR^1R^2$ | $R^4$ | Schmelzpunkt (°C) |
|---|---|---|---|---|
| 235 | $SO_2-nC_8H_{17}$ | $NH_2$ | COOH | |
| 236 | $SO_2-nC_9H_{21}$ | $NH_2$ | COOH | |
| 237 | $SO_2-nC_{10}H_{21}$ | $NH_2$ | COOH | |
| 238 | $SO_2-nC_{11}H_{23}$ | $NH_2$ | COOH | |
| 239 | $SO_2-nC_{12}H_{25}$ | $NH_2$ | COOH | |
| 240 | $SO_2-nC_{13}H_{27}$ | $NH_2$ | COOH | |
| 241 | $SO_2-nC_{14}H_{29}$ | $NH_2$ | COOH | |
| 242 | $SO_2-nC_{15}H_{31}$ | $NH_2$ | COOH | |
| 243 | $SO_2-nC_{16}H_{33}$ | $NH_2$ | COOH | |
| 244 | $SO_2-nC_{17}H_{35}$ | $NH_2$ | COOH | |
| 245 | $SO_2-nC_{18}H_{37}$ | $NH_2$ | COOH | |
| 246 | $SO_2-nC_{19}H_{39}$ | $NH_2$ | COOH | |
| 247 | $SO_2-nC_{20}H_{41}$ | $NH_2$ | COOH | |
| 248 | $SO_2$-Cyclopropyl | $NH_2$ | COOH | |
| 249 | $SO_2$-Cyclopentyl | $NH_2$ | COOH | |
| 250 | $SO_2$-Cyclohexyl | $NH_2$ | COOH | |
| 251 | $SO_2$-Cycloheptyl | $NH_2$ | COOH | |
| 252 | $SO_2$-Cyclooctyl | $NH_2$ | COOH | |
| 253 | $SO_2-C_6H_5$ | $NH_2$ | COOH | |
| 254 | $SO_2-CH_2C_6H_5$ | $NH_2$ | COOH | |
| 255 | $SO_2-(CH_2)_2-C_6H_5$ | $NH_2$ | COOH | |
| 256 | $SO_2-CH_2CH_2OH$ | $NH_2$ | COOH | |
| 257 | $SO_2-CH_2-CH=CH_2$ | $NH_2$ | COOH | |
| 258 | $SO_2-CH_2-C\equiv CH$ | $NH_2$ | COOH | |
| 259 | $S-CH_2-C(CH_3)=CH_2$ | $NH_2$ | $COOCH_3$ | |
| 260 | $SO_2-CH_3$ | $NH_2$ | $COOCH_3$ | |
| 261 | $SO_2-C_2H_5$ | $NH_2$ | $COOCH_3$ | |
| 262 | $SO_2-nC_3H_7$ | $NH_2$ | $COOCH_3$ | |
| 263 | $SO_2-isoC_3H_7$ | $NH_2$ | $COOCH_3$ | |
| 264 | $SO_2-nC_4H_9$ | $NH_2$ | $COOCH_3$ | |
| 265 | $SO_2-isoC_4H_9$ | $NH_2$ | $COOCH_3$ | |
| 266 | $SO_2-secC_4H_9$ | $NH_2$ | $COOCH_3$ | |
| 267 | $SO_2-tertC_4H_9$ | $NH_2$ | $COOCH_3$ | |
| 268 | $SO_2-nC_5H_{11}$ | $NH_2$ | $COOCH_3$ | |
| 269 | $SO_2-neoC_5H_{11}$ | $NH_2$ | $COOCH_3$ | |
| 270 | $SO_2-nC_6H_{13}$ | $NH_2$ | $COOCH_3$ | |
| 271 | $SO_2-nC_7H_{15}$ | $NH_2$ | $COOCH_3$ | |
| 272 | $SO_2-nC_8H_{17}$ | $NH_2$ | $COOCH_3$ | |
| 273 | $SO_2-nC_9H_{19}$ | $NH_2$ | $COOCH_3$ | |

| Bsp.Nr. | $R^3$ | $NR^1R^2$ | $R^4$ | Schmelzpunkt (°C) |
|---|---|---|---|---|
| 274 | $SO_2-nC_{10}H_{21}$ | $NH_2$ | $COOCH_3$ | |
| 275 | $SO_2-nC_{11}H_{23}$ | $NH_2$ | $COOCH_3$ | |
| 276 | $SO_2-nC_{12}H_{25}$ | $NH_2$ | $COOCH_3$ | |
| 277 | $SO_2-nC_{13}H_{27}$ | $NH_2$ | $COOCH_3$ | |
| 278 | $SO_2-nC_{14}H_{29}$ | $NH_2$ | $COOCH_3$ | |
| 279 | $SO_2-nC_{15}H_{31}$ | $NH_2$ | $COOCH_3$ | |
| 280 | $SO_2-nC_{16}H_{33}$ | $NH_2$ | $COOCH_3$ | |
| 281 | $SO_2-nC_{17}H_{35}$ | $NH_2$ | $COOCH_3$ | |
| 282 | $SO_2-nC_{18}H_{37}$ | $NH_2$ | $COOCH_3$ | |
| 283 | $SO_2-nC_{19}H_{39}$ | $NH_2$ | $COOCH_3$ | |
| 284 | $SO_2-nC_{20}H_{41}$ | $NH_2$ | $COOCH_3$ | |
| 285 | $SO_2$-Cyclopropyl | $NH_2$ | $COOCH_3$ | |
| 286 | $SO_2$-Cyclopentyl | $NH_2$ | $COOCH_3$ | |
| 287 | $SO_2$-Cyclohexyl | $NH_2$ | $COOCH_3$ | |
| 288 | $SO_2$-Cycloheptyl | $NH_2$ | $COOCH_3$ | |
| 289 | $SO_2$-Cyclooctyl | $NH_2$ | $COOCH_3$ | |
| 290 | $SO_2-C_6H_5$ | $NH_2$ | $COOCH_3$ | |
| 291 | $SO_2-CH_2C_6H_5$ | $NH_2$ | $COOCH_3$ | |
| 292 | $SO_2-(CH_2)_2-C_6H_5$ | $NH_2$ | $COOCH_3$ | |
| 293 | $SO_2-CH_2CH_2OH$ | $NH_2$ | $COOCH_3$ | |
| 294 | $SO_2-CH_2-CH=CH_2$ | $NH_2$ | $COOCH_3$ | |
| 295 | $SO_2-CH_2-C\equiv CH$ | $NH_2$ | $COOCH_3$ | |
| 296 | $S-CH_2COOH$ | $NH_2$ | CHO | |
| 297 | $S-CH_2COOCH_3$ | $NH_2$ | CHO | |
| 298 | $S-CH_2COOC_2H_5$ | $NH_2$ | CHO | |
| 299 | $S-CH_2COOH$ | $NH_2$ | CN | |
| 300 | $S-CH_2COOCH_3$ | $NH_2$ | CN | |
| 301 | $S-CH_2COOC_2H_5$ | $NH_2$ | CN | |
| 302 | $S-C_2H_5$ | $NH_2$ | $CH=C(CN)_2$ | |
| 303 | $S-C_6H_5$ | $NH_2$ | $CH=C(CN)_2$ | |
| 304 | $S-CH_2C_6H_5$ | $NH_2$ | $CH=C(CN)_2$ | |
| 305 | $SO_2-C_6H_5$ | $NH_2$ | $CH=C(CN)_2$ | |
| 306 | $S-C_2H_5$ | $NH_2$ | $CH=C(CN)(COOCH_3)$ | |
| 307 | $S-C_6H_5$ | $NH_2$ | $CH=C(CN)(COOCH_3)$ | |
| 308 | $S-CH_2C_6H_5$ | $NH_2$ | $CH=C(CN)(COOCH_3)$ | |
| 309 | $SO_2-C_6H_5$ | $NH_2$ | $CH=C(CN)(COOCH_3)$ | |
| 310 | $S-nC_4H_9$ | $N=CH-N(CH_3)_2$ | CHO | |
| 311 | $S-C_6H_5$ | $N=CH-N(CH_3)_2$ | CHO | |
| 312 | $S-CH_2C_6H_5$ | $N=CH-N(CH_3)_2$ | CHO | |

| Bsp. Nr. | $R^3$ | $NR^1R^2$ | $R^4$ | Schmelzpunkt (°C) |
|---|---|---|---|---|
| 313 | $S-C_2H_5$ | $N=CH-N(CH_3)_2$ | CHO | |
| 314 | SH | $N=CH-N(CH_3)_2$ | CHO | |
| 315 | $SO_2-C_6H_5$ | $N=CH-N(CH_3)_2$ | CHO | |
| 316 | $S-nC_4H_9$ | $N=CH-N(CH_3)_2$ | CN | |
| 317 | $S-C_6H_5$ | $N=CH-N(CH_3)_2$ | CN | |
| 318 | $S-CH_2C_6H_5$ | $N=CH-N(CH_3)_2$ | CN | |
| 319 | $S-C_2H_5$ | $N=CH-N(CH_3)_2$ | CN | |
| 320 | SH | $N=CH-N(CH_3)_2$ | CN | |
| 321 | $SO_2-C_6H_5$ | $N=CH-N(CH_3)_2$ | CN | |
| 322 | $SO_2-(CH_2)_3C_6H_5$ | $NH_2$ | CHO | |
| 323 | $S-(CH_2)_3C_6H_5$ | $NH_2$ | CHO | |
| 324 | $S-CH(CH_3)C_6H_5$ | $NH_2$ | CHO | |
| 325 | $S-CH(C_2H_5)C_6H_5$ | $NH_2$ | CHO | |
| 326 | $S-C(CH_3)_2C_6H_5$ | $NH_2$ | CHO | |
| 327 | $SO_2-(CH_2)_3C_6H_5$ | $NH_2$ | CN | |
| 328 | $S-(CH_2)_3C_6H_5$ | $NH_2$ | CN | |
| 329 | $S-CH(CH_3)C_6H_5$ | $NH_2$ | CN | |
| 330 | $S-CH(C_2H_5)C_6H_5$ | $NH_2$ | CN | |
| 331 | $S-C(CH_3)_2C_6H_5$ | $NH_2$ | CN | |
| 332 | $S-C_6H_5$ | N=CH-morpholino | CHO | |
| 333 | $S-CH_2C_6H_5$ | N=CH-pyrrolidino | CHO | |
| 334 | $SO_2-C_6H_5$ | N=CH-piperidino | CHO | |
| 335 | $S-C_2H_5$ | N=CH-piperazino | CN | |
| 336 | $S-C_6H_5$ | N=CH-thiomorpholino | CHO | |
| 337 | $S-CH_2C_6H_5$ | N=CH-hexamethylenimino | CN | |
| 338 | $S-C_6H_5$ | $NH_2$ | $COCH_3$ | |
| 339 | $S-C_2H_5$ | $NH_2$ | $COCH_3$ | |
| 340 | $S-nC_4H_9$ | $NH_2$ | $COCH_3$ | |
| 341 | $S-CH_2C_6H_5$ | $NH_2$ | $COCH_3$ | |
| 342 | $SO_2-C_6H_5$ | $NH_2$ | $COCH_3$ | |
| 343 | $S-C_6H_5$ | $NH_2$ | $CO_2C_2H_5$ | |
| 344 | $S-C_2H_5$ | $NH_2$ | $COC_2H_5$ | |
| 345 | $S-nC_4H_9$ | $NH_2$ | $COC_2H_5$ | |
| 346 | $S-CH_2C_6H_5$ | $NH_2$ | $COC_2H_5$ | |
| 347 | $SO_2-C_6H_5$ | $NH_2$ | $COC_2H_5$ | |
| 348 | $S-C_6H_5$ | $NH_2$ | $CO-nC_3H_7$ | |
| 349 | $S-C_2H_5$ | $NH_2$ | $CO-nC_4H_9$ | |

| Bsp. Nr. | $R^3$ | $NR^1R^2$ | $R^4$ | Schmelzpunkt (°C) |
|---|---|---|---|---|
| 350 | $S-nC_4H_9$ | $NH_2$ | $CO-nC_5H_{11}$ | |
| 351 | $S-CH_2C_6H_5$ | $NH_2$ | $CO-nC_6H_{13}$ | |
| 352 | $SO_2-C_6H_5$ | $NH_2$ | $CO-nC_6H_{13}$ | |
| 353 | $S-C_6H_5$ | $NH_2$ | $COC_6H_5$ | |
| 354 | $S-C_2H_5$ | $NH_2$ | $COC_6H_5$ | |
| 355 | $S-nC_4H_9$ | $NH_2$ | $COC_6H_5$ | |
| 356 | $S-CH_2C_6H_5$ | $NH_2$ | $COC_6H_5$ | |
| 357 | $SO_2-C_6H_5$ | $NH_2$ | $COC_6H_5$ | |
| 358 | $S-C_2H_5$ | $NH_2$ | $COOC_2H_5$ | |
| 359 | $S-nC_4H_9$ | $NH_2$ | $COOC_2H_5$ | |
| 360 | $S-nC_6H_{13}$ | $NH_2$ | $COOC_2H_5$ | |
| 361 | $S-C_6H_5$ | $NH_2$ | $COOC_2H_5$ | |
| 362 | $S-CH_2C_6H_5$ | $NH_2$ | $COOC_2H_5$ | |
| 363 | $SO_2-C_6H_5$ | $NH_2$ | $COOC_2H_5$ | |
| 364 | $S-C_2H_5$ | $NH_2$ | $COO-nC_3H_7$ | |
| 365 | $S-nC_4H_9$ | $NH_2$ | $COO-nC_3H_7$ | |
| 366 | $S-nC_6H_{13}$ | $NH_2$ | $COO-nC_3H_7$ | |
| 367 | $S-C_6H_5$ | $NH_2$ | $COO-nC_3H_7$ | |
| 368 | $S-CH_2C_6H_5$ | $NH_2$ | $COO-nC_3H_7$ | |
| 369 | $SO_2-C_6H_5$ | $NH_2$ | $COO-nC_3H_7$ | |
| 370 | $S-C_2H_5$ | $NH_2$ | $COO-nC_4H_9$ | |
| 371 | $S-nC_4H_9$ | $NH_2$ | $COO-nC_4H_9$ | |
| 372 | $S-nC_6H_{13}$ | $NH_2$ | $COO-nC_4H_9$ | |
| 373 | $S-C_6H_5$ | $NH_2$ | $COO-nC_4H_9$ | |
| 374 | $S-CH_2C_6H_5$ | $NH_2$ | $COO-nC_4H_9$ | |
| 375 | $SO_2-C_6H_5$ | $NH_2$ | $COO-nC_4H_9$ | |
| 376 | $S-C_2H_5$ | $NH_2$ | $COO-nC_5H_{11}$ | |
| 377 | $S-nC_4H_9$ | $NH_2$ | $COO-nC_5H_{11}$ | |
| 378 | $S-nC_6H_{13}$ | $NH_2$ | $COO-nC_5H_{11}$ | |
| 379 | $S-C_6H_5$ | $NH_2$ | $COO-nC_5H_{11}$ | |
| 380 | $S-CH_2C_6H_5$ | $NH_2$ | $COO-nC_5H_{11}$ | |
| 381 | $SO_2-C_6H_5$ | $NH_2$ | $COO-nC_5H_{11}$ | |
| 382 | $S-C_2H_5$ | $NH_2$ | $COO-nC_6H_{13}$ | |
| 383 | $S-nC_4H_9$ | $NH_2$ | $COO-nC_6H_{13}$ | |
| 384 | $S-nC_6H_{13}$ | $NH_2$ | $COO-nC_6H_{13}$ | |
| 385 | $S-C_6H_5$ | $NH_2$ | $COO-nC_6H_{13}$ | |
| 386 | $S-CH_2C_6H_5$ | $NH_2$ | $COO-nC_6H_{13}$ | |
| 387 | $SO_2-C_6H_5$ | $NH_2$ | $COO-nC_6H_{13}$ | |
| 388 | $S-C_2H_5$ | $NH_2$ | $COOC_6H_5$ | |

| Bsp. Nr. | $R^3$ | $NR^1R^2$ | $R^4$ | Schmelzpunkt (°C) |
|---|---|---|---|---|
| 389 | $S-nC_4H_9$ | $NH_2$ | $COOC_6H_5$ | |
| 390 | $S-nC_6H_{13}$ | $NH_2$ | $COOC_6H_5$ | |
| 391 | $S-C_6H_5$ | $NH_2$ | $COOC_6H_5$ | |
| 392 | $S-CH_2C_6H_5$ | $NH_2$ | $COOC_6H_5$ | |
| 393 | $SO_2-C_6H_5$ | $NH_2$ | $COOC_6H_5$ | |
| 394 | $S-C_2H_5$ | $NH_2$ | $CONH_2$ | |
| 395 | $S-nC_4H_9$ | $NH_2$ | $CONH_2$ | |
| 396 | $S-nC_6H_{13}$ | $NH_2$ | $CONH_2$ | |
| 397 | $S-C_6H_5$ | $NH_2$ | $CONH_2$ | |
| 398 | $S-CH_2C_6H_5$ | $NH_2$ | $CONH_2$ | |
| 399 | $SO_2-C_6H_5$ | $NH_2$ | $CONH_2$ | |
| 400 | $S-C_6H_5$ | $NH_2$ | $C(CH_3)=C(CN)_2$ | |
| 401 | $SO_2-C_6H_5$ | $NH_2$ | $C(C_6H_5)=C(CN)_2$ | |
| 402 | $S-C_2H_5$ | $NH_2$ | $CON(CH_3)_2$ | |
| 403 | $S-nC_4H_9$ | $NH_2$ | $CON(CH_3)_2$ | |
| 404 | $S-nC_6H_{13}$ | $NH_2$ | $CON(CH_3)_2$ | |
| 405 | $S-C_6H_5$ | $NH_2$ | $CON(CH_3)_2$ | |
| 406 | $S-CH_2C_6H_5$ | $NH_2$ | $CON(CH_3)_2$ | |
| 407 | $SO_2-C_6H_5$ | $NH_2$ | $CON(CH_3)_2$ | |
| 408 | $S-C_2H_5$ | $NH_2$ | $CON(C_2H_5)_2$ | |
| 409 | $S-nC_4H_9$ | $NH_2$ | $CON(C_2H_5)_2$ | |
| 410 | $S-nC_6H_{13}$ | $NH_2$ | $CON(C_2H_5)_2$ | |
| 411 | $S-C_6H_5$ | $NH_2$ | $CON(C_2H_5)_2$ | |
| 412 | $S-CH_2C_6H_5$ | $NH_2$ | $CON(C_2H_5)_2$ | |
| 413 | $SO_2-C_6H_5$ | $NH_2$ | $CON(C_2H_5)_2$ | |
| 414 | $S-C_2H_5$ | $NH_2$ | $CON(C_3H_7)_2$ | |
| 415 | $S-nC_4H_9$ | $NH_2$ | $CON(C_3H_7)_2$ | |
| 416 | $S-nC_6H_{13}$ | $NH_2$ | $CON(C_3H_7)_2$ | |
| 417 | $S-C_6H_5$ | $NH_2$ | $CON(C_3H_7)_2$ | |
| 418 | $S-CH_2C_6H_5$ | $NH_2$ | $CON(C_3H_7)_2$ | |
| 419 | $SO_2-C_6H_5$ | $NH_2$ | $CON(C_3H_7)_2$ | |
| 420 | $S-C_2H_5$ | $NH_2$ | $CON(C_4H_9)_2$ | |
| 421 | $S-nC_4H_9$ | $NH_2$ | $CON(C_5H_{11})_2$ | |
| 422 | $S-nC_6H_{13}$ | $NH_2$ | $CON(C_6H_{11})_2$ | |
| 423 | $S-C_6H_5$ | $NH_2$ | $CON(CH_3)(C_6H_5)$ | |
| 424 | $S-CH_2C_6H_5$ | $NH_2$ | $CON(C_2H_5)(C_6H_5)$ | |
| 425 | $SO_2-C_6H_5$ | $NH_2$ | $CON(CH_3)(C_2H_5)$ | |
| 426 | $S-C_6H_5$ | $NH_2$ | $CON(n-C_3H_7)(C_6H_5)$ | |
| 427 | $SO_2-C_6H_5$ | $NH_2$ | $CON(n-C_4H_9)(C_6H_5)$ | |

| Bsp. Nr. | $R^3$ | $NR^1R^2$ | $R^4$ | Schmelzpunkt (°C) |
|---|---|---|---|---|
| 428 | $SO_2-C_6H_5$ | $NH_2$ | $CON(n-C_6H_{13})(C_6H_5)$ | |
| 429 | $S-C_2H_5$ | $NH_2$ | $CH=C(CN)(NO_2)$ | |
| 430 | $S-nC_4H_9$ | $NH_2$ | $CH=C(CN)(NO_2)$ | |
| 431 | $S-nC_6H_{13}$ | $NH_2$ | $CH=C(CN)(CONH_2)$ | |
| 432 | $S-C_6H_5$ | $NH_2$ | $CH=C(CN)(CONH_2)$ | |
| 433 | $S-CH_2C_6H_5$ | $NH_2$ | $CH=C(CN)(CONH_2)$ | |
| 434 | $SO_2-C_6H_5$ | $NH_2$ | $CH=C(CN)(CONH_2)$ | |
| 435 | $S-C_2H_5$ | $NH_2$ | $C(CH_3)=C(CN)_2$ | |
| 436 | $S-nC_4H_9$ | $NH_2$ | $C(CH_3)=C(CN)(NO_2)$ | |
| 437 | $S-nC_6H_{13}$ | $NH_2$ | $C(C_6H_5)=C(CN)_2$ | |
| 438 | $S-C_6H_5$ | $NH_2$ | $C(C_6H_5)=C(CN)_2$ | |
| 439 | $S-CH_2C_6H_5$ | $NH_2$ | $C(C_6H_5)=C(CN)_2$ | |
| 440 | $SO_2-C_6H_5$ | $NH_2$ | $C(C_6H_5)=C(CN)_2$ | |
| 441 | $S-C_6H_4-p-CH_3$ | $NH_2$ | CHO | 204,5 - 205 |
| 442 | $S-C_2H_5$ | $NH_2$ | CO-Morpholino | |
| 443 | $S-nC_4H_9$ | $NH_2$ | CO-Morpholino | |
| 444 | $S-nC_6H_{13}$ | $NH_2$ | CO-Morpholino | |
| 445 | $S-C_6H_5$ | $NH_2$ | CO-Morpholino | |
| 446 | $S-CH_2C_6H_5$ | $NH_2$ | CO-Morpholino | |
| 447 | $SO_2-C_6H_5$ | $NH_2$ | CO-Morpholino | |
| 448 | $S-C_2H_5$ | $NH_2$ | CO-Piperidino | |
| 449 | $S-nC_4H_9$ | $NH_2$ | CO-Piperidino | |
| 450 | $S-nC_6H_{13}$ | $NH_2$ | CO-Piperidino | |
| 451 | $S-C_6H_5$ | $NH_2$ | CO-Piperidino | |
| 452 | $S-CH_2C_6H_5$ | $NH_2$ | CO-Piperidino | |
| 453 | $SO_2-C_6H_5$ | $NH_2$ | CO-Piperidino | |
| 454 | $S-C_2H_5$ | $NH_2$ | CO-Pyrrolidino | |
| 455 | $S-nC_4H_9$ | $NH_2$ | CO-Pyrrolidino | |
| 456 | $S-nC_6H_{13}$ | $NH_2$ | CO-Pyrrolidino | |
| 457 | $S-C_6H_5$ | $NH_2$ | CO-Pyrrolidino | |
| 458 | $S-CH_2C_6H_5$ | $NH_2$ | CO-Pyrrolidino | |
| 459 | $SO_2-C_6H_5$ | $NH_2$ | CO-Pyrrolidino | |
| 460 | $S-C_2H_5$ | $NH_2$ | CO-Thiomorpholino | |
| 461 | $S-nC_4H_9$ | $NH_2$ | CO-Thiomorpholino | |
| 462 | $S-nC_6H_{13}$ | $NH_2$ | CO-Thiomorpholino | |
| 463 | $S-C_6H_5$ | $NH_2$ | CO-Hexamethylenimino | |
| 464 | $S-CH_2C_6H_5$ | $NH_2$ | CO-Hexamethylenimino | |
| 465 | $SO_2-C_6H_5$ | $NH_2$ | CO-Hexamethylenimino | |
| 466 | $S-C_2H_5$ | $NH_2$ | CO-(N-Methyl-piperazino) | |

| Bsp. Nr. | $R^3$ | $NR^1R^2$ | $R^4$ | Schmelzpunkt (°C) |
|---|---|---|---|---|
| 467 | $S{-}nC_4H_9$ | $NH_2$ | CO–(N-Methyl-piperazino) | |
| 468 | $S{-}nC_6H_{13}$ | $NH_2$ | CO–(N-Ethyl-piperazino) | |
| 469 | $S{-}C_6H_5$ | $NH_2$ | CO–(N-Ethyl-piperazino) | |
| 470 | $S{-}CH_2C_6H_5$ | $NH_2$ | CO–(N-Ethyl-piperazino) | |
| 471 | $SO_2{-}C_6H_5$ | $NH_2$ | CO–(N-Ethyl-piperazino) | |
| 472 | $S{-}C_2H_5$ | $NH_2$ | COO–Cyclopropyl | |
| 473 | $S{-}nC_4H_9$ | $NH_2$ | COO–Cyclopropyl | |
| 474 | $S{-}nC_6H_{13}$ | $NH_2$ | COO–Cyclopentyl | |
| 475 | $S{-}C_6H_5$ | $NH_2$ | COO–Cyclopentyl | |
| 476 | $S{-}CH_2C_6H_5$ | $NH_2$ | COO–Cyclohexyl | |
| 477 | $SO_2{-}C_6H_5$ | $NH_2$ | COO–Cyclohexyl | |

Beispiel 478

8,1 g 2-Amino-4-chlor-5-formylthiazol wurden in 40 ml N,N-Dimethylformamid gelöst. Hierzu wurden bei 35 bis 40°C 9,9 ml Piperidin zugetropft. Das Reaktionsgemisch wurde 4 Stunden bei Raumtemperatur nachgerührt und anschließend auf 150 ml Eiswasser gegossen. Der Niederschlag wurde abgesaugt, mit Wasser nachgewaschen und unter vermindertem Druck bei 50°C getrocknet. Man erhielt 6,3 g (60 % d. Th.) der Verbindung der Formel

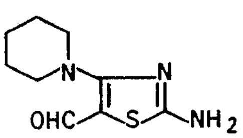

vom Schmelzpunkt 160°C (Zersetzung). Die NMR-, IR-, UV- und Massenspektren sowie die Elementaranalyse stehen mit der angegebenen Strukturformel im Einklang.

Beispiel 479

8 g 2-Amino-4-chlor-5-cyanothiazol wurden in 50 ml N,N-Dimethylformamid gelöst. Hierzu wurden bei 35°C 7,9 ml 70 gew.-%ige wäßrige Ethylamin-Lösung zugetropft. Das Reaktionsgemisch wurde 1 Stunde bei Raumtemperatur gerührt. Anschließend wurde nochmals 1 ml 70 gew.-%ige wäßriger Ethylamin-Lösung zugegeben. Nach weiteren 0,5 Stunden Rühren bei Raumtemperatur wurde das Reaktionsgemisch auf 400 ml Eiswasser und 5 ml Salzsäure (M 100) gegeben. Der Niederschlag wurde abgesaugt, mit Wasser nachgewaschen und unter vermindertem Druck bei 50°C getrocknet. Man erhielt 4,0 g (48 % d. Th.) der Verbindung der Formel

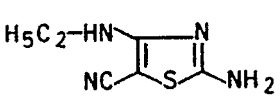

vom Schmelzpunkt 171 bis 174°C. Die NMR-, IR-, UV- und Massenspektren sowie die Daten der Elementaranalyse stehen mit der oben angegebenen Strukturformel im Einklang.

In analoger Weise lassen sich die in der folgenden Tabelle 2 aufgeführten Verbindungen der Formel

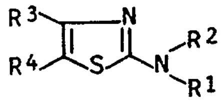

darstellen.

Tabelle 2

| Bsp-Nr. | $R^3$ | $NR^1R^2$ | $R^4$ | Schmelzpunkt (°C) |
|---|---|---|---|---|
| 480 | $NH-CH_3$ | $NH_2$ | CN | |
| 481 | $NH-nC_3H_7$ | $NH_2$ | CN | 121-23 |
| 482 | $NH-isoC_3H_7$ | $NH_2$ | CN | 134-36 |
| 483 | $NH-nC_4H_9$ | $NH_2$ | CN | |
| 484 | $NH-secC_4H_9$ | $NH_2$ | CN | |
| 485 | $NH-isoC_4H_9$ | $NH_2$ | CN | 203-05 |
| 486 | $NH-tertC_4H_9$ | $NH_2$ | CN | 152-53 |
| 487 | $NH-nC_5H_{11}$ | $NH_2$ | CN | |
| 488 | $NH-isoC_5H_{11}$ | $NH_2$ | CN | |
| 489 | $NH-CH(CH_3)-CH(CH_3)_2$ | $NH_2$ | CN | |
| 490 | $NH-neoC_5H_{11}$ | $NH_2$ | CN | 117-19 |
| 491 | $NH-nC_6H_{13}$ | $NH_2$ | CN | |
| 492 | $NH-nC_7H_{15}$ | $NH_2$ | CN | |
| 493 | $NH-nC_8H_{17}$ | $NH_2$ | CN | |
| 494 | $NH-nC_9H_{19}$ | $NH_2$ | CN | |
| 495 | $NH-nC_{10}H_{21}$ | $NH_2$ | CN | |
| 496 | $NH-nC_{12}H_{25}$ | $NH_2$ | CN | |
| 497 | $NH-nC_{13}H_{27}$ | $NH_2$ | CN | |
| 498 | $NH-nC_{16}H_{33}$ | $NH_2$ | CN | |
| 499 | $NH-nC_{18}H_{37}$ | $NH_2$ | CN | |
| 500 | $NH-CH(CH_3)CH_2CH_2CH_3$ | $NH_2$ | CN | |
| 501 | $NH-CH(CH_3)-(CH_2)_3CH_3$ | $NH_2$ | CN | |
| 502 | $NH-CH(CH_3)-(CH_2)_2-CH(CH_3)_2$ | $NH_2$ | CN | |
| 503 | $NH-CH(CH_3)-(CH_2)_5-CH_3$ | $NH_2$ | CN | |
| 504 | $NH-CH(C_2H_5)-(CH_2)_4-CH_3$ | $NH_2$ | CN | |
| 505 | $NH-CH(CH_3)-(CH_2)_3-CH(CH_3)_2$ | $NH_2$ | CN | |
| 506 | (R)-(-)-$NH-CH(CH_3)$- -Cyclohexyl | $NH_2$ | CN | |
| 507 | (S)-(+)-$NH-CH(CH_3)$- -Cyclohexyl | $NH_2$ | CN | |
| 508 | $NH-CH_2CH_2$-Cyclohexyl | $NH_2$ | CN | |
| 509 | $NH-(CH_2)_2-OH$ | $NH_2$ | CN | |
| 510 | $NH-(CH_2)_2-OCH_3$ | $NH_2$ | CN | |
| 511 | $NH-(CH_2)_2-OC_2H_5$ | $NH_2$ | CN | |
| 512 | $NH-(CH_2)_2-OC_6H_5$ | $NH_2$ | CN | |
| 513 | $NH-(CH_2)_2-O-(CH_2)_2OH$ | $NH_2$ | CN | |

| Bsp-Nr. | $R^3$ | $NR^1R^2$ | $R^4$ | Schmelzpunkt (°C) |
|---|---|---|---|---|
| 514 | $NH-(CH_2)_2-NH-(CH_2)_2OH$ | $NH_2$ | CN | |
| 515 | $NH-(CH_2)_2-NH_2$ | $NH_2$ | CN | |
| 516 | $NH-(CH_2)_2-N(CH_3)_2$ | $NH_2$ | CN | |
| 517 | $NH-(CH_2)_2-N(C_2H_5)_2$ | $NH_2$ | CN | |
| 518 | $NH-(CH_2)_2-N(iso-C_3H_7)_2$ | $NH_2$ | CN | |
| 519 | $NH-(CH_2)_2$-Piperazino | $NH_2$ | CN | |
| 520 | $NH-(CH_2)_3-OH$ | $NH_2$ | CN | |
| 521 | $NH-(CH_2)_3-OCH_3$ | $NH_2$ | CN | |
| 522 | $NH-(CH_2)_3-OC_2H_5$ | $NH_2$ | CN | |
| 523 | $NH-(CH_2)_3-OCH(CH_3)_2$ | $NH_2$ | CN | |
| 524 | $NH-(CH_2)_3-OC_6H_5$ | $NH_2$ | CN | |
| 525 | $NH-(CH_2)_3-O-CH_2C_6H_5$ | $NH_2$ | CN | |
| 526 | $NH-(CH_2)_3-O-(CH_2)_2-O-CH_3$ | $NH_2$ | CN | |
| 527 | $NH-(CH_2)_2-O-(CH_2)_2-O-C_2H_5$ | $NH_2$ | CN | |
| 528 | $NH-(CH_2)_3-O-(CH_2)_2-O-C_6H_5$ | $NH_2$ | CN | |
| 529 | $NH-(CH_2)_3-NH_2$ | $NH_2$ | CN | |
| 530 | $NH-(CH_2)_3-N(CH_3)_2$ | $NH_2$ | CN | |
| 531 | $NH-(CH_2)_3-N(C_2H_5)_2$ | $NH_2$ | CN | |
| 532 | $NH-(CH_2)_3-N(n-C_4H_9)_2$ | $NH_2$ | CN | |
| 533 | $NH-(CH_2)_3$-Morpholino | $NH_2$ | CN | |
| 534 | $NH-(CH_2)_4-OH$ | $NH_2$ | CN | |
| 535 | $NH-(CH_2)_5-NH_2$ | $NH_2$ | CN | |
| 536 | $NH-(CH_2)_6-OH$ | $NH_2$ | CN | |
| 537 | $NH-(CH_2)_6-NH_2$ | $NH_2$ | CN | |
| 538 | $NH-(CH_2)_7-NH_2$ | $NH_2$ | CN | |
| 539 | $NH-(CH_2)_8-NH_2$ | $NH_2$ | CN | |
| 540 | $NH-(CH_2)_9-NH_2$ | $NH_2$ | CN | |
| 541 | $NH-(CH_2)_{10}-NH_2$ | $NH_2$ | CN | |
| 542 | $NH-(CH_2)_{12}-NH_2$ | $NH_2$ | CN | |
| 543 | $NH-CH(CH_3)-(CH_2)_3-N(C_2H_5)_2$ x HCl | $NH_2$ | CN | |
| 544 | $NH-CH_2-CH(CH_3)-OH$ | $NH_2$ | CN | |
| 545 | $NH-CH(CH_3)-CH_2-OH$ | $NH_2$ | CN | |
| 546 | $NH-CH_2$-Thien-2-yl | $NH_2$ | CN | |
| 547 | $NH-C(CH_3)_2-CH_2-OH$ | $NH_2$ | CN | |
| 548 | $NH-C(CH_3)-(CH_2-OH)_2$ | $NH_2$ | CN | |
| 549 | $NH-CH(C_2H_5)-CH_2-OH$ | $NH_2$ | CN | |
| 550 | $NH-(CH_2)_2-COOH$ | $NH_2$ | CN | |

| Bsp-Nr. | $R^3$ | $NR^1R^2$ | $R^4$ | Schmelzpunkt (°C) |
|---|---|---|---|---|
| 551 | $NH-(CH_2)_5-COOH$ | $NH_2$ | CN | |
| 552 | $NH-(CH_2)_{10}-COOH$ | $NH_2$ | CN | |
| 553 | $NH-C_6H_5$ | $NH_2$ | CN | |
| 554 | $NH-C_6H_4-(2)-CH_3$ | $NH_2$ | CN | |
| 555 | $NH-C_6H_4-(3)-CH_3$ | $NH_2$ | CN | |
| 556 | $NH-C_6H_4-(4)-CH_3$ | $NH_2$ | CN | |
| 557 | $NH-C_6H_5-(2)-C_2H_5$ | $NH_2$ | CN | |
| 558 | $NH-C_6H_4-(4)-C_2H_5$ | $NH_2$ | CN | |
| 559 | $NH-C_6H_4-(2)-CF_3$ | $NH_2$ | CN | |
| 560 | $NH-C_6H_4-(3)-CF_3$ | $NH_2$ | CN | |
| 561 | $NH-C_6H_4-(4)-CF_3$ | $NH_2$ | CN | |
| 562 | $NH-C_6H_5-(2)-OH$ | $NH_2$ | CN | |
| 563 | $NH-C_6H_5-(3)-OH$ | $NH_2$ | CN | |
| 564 | $NH-C_6H_5-(4)-OH$ | $NH_2$ | CN | |
| 565 | $NH-C_6H_4-(2)-OCH_3$ | $NH_2$ | CN | |
| 566 | $NH-C_6H_4-(3)-OCH_3$ | $NH_2$ | CN | |
| 567 | $NH-C_6H_4-(4)-OCH_3$ | $NH_2$ | CN | |
| 568 | $NH-C_6H_4-(2)-OC_2H_5$ | $NH_2$ | CN | |
| 569 | $NH-C_6H_4-(3)-OC_2H_5$ | $NH_2$ | CN | |
| 570 | $NH-C_6H_4-(4)-OC_2H_5$ | $NH_2$ | CN | |
| 571 | $NH-C_6H_4-(2)-Cl$ | $NH_2$ | CN | |
| 572 | $NH-C_6H_4-(3)-Cl$ | $NH_2$ | CN | |
| 573 | $NH-C_6H_4-(4)-Cl$ | $NH_2$ | CN | |
| 574 | $NH-C_6H_4-(2)-F$ | $NH_2$ | CN | |
| 575 | $NH-C_6H_4-(3)-F$ | $NH_2$ | CN | |
| 576 | $NH-C_6H_4-(4)-F$ | $NH_2$ | CN | |
| 577 | $NH-C_6H_4-(2)-Br$ | $NH_2$ | CN | |
| 578 | $NH-C_6H_4-(3)-Br$ | $NH_2$ | CN | |
| 579 | $NH-C_6H_4-(4)-Br$ | $NH_2$ | CN | |
| 580 | $NH-C_6H_4-(2)-I$ | $NH_2$ | CN | |
| 581 | $NH-C_6H_4-(3)-I$ | $NH_2$ | CN | |
| 582 | $NH-C_6H_4-(4)-I$ | $NH_2$ | CN | |
| 583 | $NH-C_6H_4-(2)-NH_2$ | $NH_2$ | CN | |
| 584 | $NH-C_6H_4-(3)-NH_2$ | $NH_2$ | CN | |
| 585 | $NH-C_6H_4-(4)-NH_2$ | $NH_2$ | CN | |
| 586 | $NH-C_6H_4-(4)-NH-COCH_3$ | $NH_2$ | CN | |
| 587 | $NH-C_6H_4-(2)-CH_2OH$ | $NH_2$ | CN | |
| 588 | $NH-C_6H_4-(3)-CH_2OH$ | $NH_2$ | CN | |

| Bsp-Nr. | $R^3$ | $NR^1R^2$ | $R^4$ | Schmelzpunkt (°C) |
|---|---|---|---|---|
| 589 | $NH-C_6H_4-(4)-COCH_3$ | $NH_2$ | CN | |
| 590 | $NH-C_6H_4-(2)-COOH$ | $NH_2$ | CN | |
| 591 | $NH-C_6H_4-(3)-COOH$ | $NH_2$ | CN | |
| 592 | $NH-C_6H_4-(4)-COOH$ | $NH_2$ | CN | |
| 593 | $NH-C_6H_4-(2)-CONH_2$ | $NH_2$ | CN | |
| 594 | $NH-C_6H_4-(4)-CONH_2$ | $NH_2$ | CN | |
| 595 | $NH-C_6H_4-(2)-CN$ | $NH_2$ | CN | |
| 596 | $NH-C_6H_4-(3)-CN$ | $NH_2$ | CN | |
| 597 | $NH-C_6H_4-(4)-CN$ | $NH_2$ | CN | |
| 598 | $NH-C_6H_3-(2,6)-(CH_3)_2$ | $NH_2$ | CN | |
| 599 | $NH-C_6H_3-(3,5)-(CH_3)_2$ | $NH_2$ | CN | |
| 600 | $NH-C_6H_3-(2)-OH-(5)-CH_3$ | $NH_2$ | CN | |
| 601 | $NH-C_6H_3-(2)-OH-(4)-CH_3$ | $NH_2$ | CN | |
| 602 | $NH-C_6H_3-(2)-OH-(2)-CH_3$ | $NH_2$ | CN | |
| 603 | $NH-C_6H_3-(2)-OH-(5)-Cl$ | $NH_2$ | CN | |
| 604 | NH-Pyrid-2-yl | $NH_2$ | CN | |
| 605 | NH-Pyrid-3-yl | $NH_2$ | CN | |
| 606 | NH-Pyrid-4-yl | $NH_2$ | CN | |
| 607 | $NH-CH_2$-Pyrid-3-yl | $NH_2$ | CN | |
| | $NH-CH_2$-Fur-2-yl | $NH_2$ | CN | |
| 608 | $NH-CH_2-C_6H_5$ | $NH_2$ | CN | 119-22 |
| 609 | $NH-CH_2-C_6H_4-(2)-OCH_3$ | $NH_2$ | CN | |
| 610 | $NH-CH_2-C_6H_4-(4)-OCH_3$ | $NH_2$ | CN | |
| 611 | $NH-CH_2-C_6H_4-(4)-F$ | $NH_2$ | CN | |
| 612 | $NH-CH_2-C_6H_4-(2)-Cl$ | $NH_2$ | CN | |
| 613 | $NH-CH_2-C_6H_4-(4)-Cl$ | $NH_2$ | CN | |
| 614 | $NH-CH_2-C_6H_3-(3,4)-(OCH_3)_2$ | $NH_2$ | CN | |
| 615 | $NH-CH_2-CH_2-C_6H_5$ | $NH_2$ | CN | |
| 616 | $NH-CH(CH_3)-C_6H_5$ (+/-) | $NH_2$ | CN | |
| 617 | $NH-(CH_2)_3-C_6H_5$ | $NH_2$ | CN | |
| 618 | $NH-CH(CH_3)-(CH_2)_2-C_6H_5$ | $NH_2$ | CN | |
| 619 | NH-Cyclopropyl | $NH_2$ | CN | |
| 620 | NH-Cyclopentyl | $NH_2$ | CN | |
| 621 | NH-Cyclohexyl | $NH_2$ | CN | |
| 622 | NH-Cycloheptyl | $NH_2$ | CN | |
| 623 | NH-Cyclooctyl | $NH_2$ | CN | |
| 624 | NH-Cyclododecyl | $NH_2$ | CN | |
| 625 | $NH-CH_2-CH=CH_2$ | $NH_2$ | CN | |

| Bsp-Nr. | $R^3$ | $NR^1R^2$ | $R^4$ | Schmelzpunkt (°C) |
|---|---|---|---|---|
| 626 | $NH-CH_2-C{\equiv}CH$ | $NH_2$ | CN | |
| 627 | $N(CH_3)_2$ | $NH_2$ | CN | |
| 628 | $N(C_2H_5)_2$ | $NH_2$ | CN | |
| 629 | $N(n-C_3H_7)_2$ | $NH_2$ | CN | |
| 630 | $N(n-C_4H_9)_2$ | $NH_2$ | CN | |
| 631 | $N(iso-C_4H_9)_2$ | $NH_2$ | CN | |
| 632 | $N(n-C_5H_{11})_2$ | $NH_2$ | CN | |
| 633 | $N(sec-C_4H_9)_2$ | $NH_2$ | CN | |
| 634 | $N(iso-C_5H_{11})_2$ | $NH_2$ | CN | |
| 635 | $N(n-C_6H_{13})_2$ | $NH_2$ | CN | |
| 636 | $N(CH(C_2H_5)-(CH_2)_4-CH_3)_2$ | $NH_2$ | CN | |
| 637 | $N(n-C_8H_{17})_2$ | $NH_2$ | CN | |
| 638 | $N(CH_2-CH{=}CH_2)_2$ | $NH_2$ | CN | |
| 639 | N(Cyclohexyl)$_2$ | $NH_2$ | CN | |
| 640 | $N(CH_3)-nC_4H_9$ | $NH_2$ | CN | |
| 641 | $N(CH_3)$-Cyclohexyl | $NH_2$ | CN | |
| 642 | $N(C_2H_5)$-Cyclohexyl | $NH_2$ | CN | |
| 643 | $N(CH_3)-(CH_2)_2-OH$ | $NH_2$ | CN | |
| 644 | $N(C_2H_5)-CH(CH_3)-CH(CH_3)_2$ | $NH_2$ | CN | |
| 645 | $N(C_2H_5)-(CH)_2-OH$ | $NH_2$ | CN | |
| 646 | $N(C_2H_5)-CH_2-CH(OH)-CH_3$ | $NH_2$ | CN | |
| 647 | $N(tert-C_4H_9)-(CH_2)_2-OH$ | $NH_2$ | CN | |
| 648 | $N(CH_3)-C_6H_5$ | $NH_2$ | CN | |
| 649 | $N(C_2H_5)-C_6H_5$ | $NH_2$ | CN | |
| 650 | $N(iso-C_3H_7)-C_6H_5$ | $NH_2$ | CN | |
| 651 | $N(nC_4H_9)-C_6H_5$ | $NH_2$ | CN | |
| 652 | $N(CH_2C_6H_5)_2$ | $NH_2$ | CN | |
| 653 | $N(CH_3)-CH_2C_6H_5$ | $NH_2$ | CN | |
| 654 | $N(C_2H_5)-CH_2C_6H_5$ | $NH_2$ | CN | |
| 655 | $N(iso-C_3H_7)-CH_2C_6H_5$ | $NH_2$ | CN | |
| 656 | $N(tert-C_4H_9)-CH_2C_6H_5$ | $NH_2$ | CN | |
| 657 | $N(CH_2C_6H_5)-(CH_2)_2-OH$ | $NH_2$ | CN | |
| 658 | $N(C_6H_5)-CH_2C_6H_5$ | $NH_2$ | CN | |
| 659 | $N(CH_2C_6H_5)-((CH_2)_2-C_6H_5)$ | $NH_2$ | CN | |
| 660 | Pyrrolidino | $NH_2$ | CN | |
| 661 | Hexamethylenimino | $NH_2$ | CN | |
| 662 | N-Methylpiperazino | $NH_2$ | CN | |
| 663 | $NH-NH_2$ | $NH_2$ | CN | |

| Bsp-Nr. | $R^3$ | $NR^1R^2$ | $R^4$ | Schmelzpunkt (°C) |
|---|---|---|---|---|
| 664 | $NH-NH-CH_3$ | $NH_2$ | CN | |
| 665 | $NH-NH-C_6H_5$ | $NH_2$ | CN | |
| 666 | $NH-N(CH_3)_2$ | $NH_2$ | CN | |
| 667 | NH-OH | $NH_2$ | CN | |
| 668 | $NH-CH_3$ | $NH_2$ | CHO | |
| 669 | $NH-C_2H_5$ | $NH_2$ | CHO | |
| 670 | $NH-nC_3H_7$ | $NH_2$ | CHO | |
| 671 | $NH-isoC_3H_7$ | $NH_2$ | CHO | |
| 672 | $NH-secC_4H_9$ | $NH_2$ | CHO | |
| 673 | $NH-nC_5H_{11}$ | $NH_2$ | CHO | |
| 674 | NH-Cyclohexyl | $NH_2$ | CHO | |
| 675 | $NH-C_6H_5$ | $NH_2$ | CHO | |
| 676 | $N(C_2H_5)_2$ | $NH_2$ | CHO | |
| 677 | $NH-CH_3$ | $N=CH-N(CH_3)_2$ | CN | |
| 678 | $NH-C_2H_5$ | $N=CH-N(CH_3)_2$ | CN | |
| 679 | $NH-CH_3$ | $NH_2$ | $CH=C(CN)_2$ | |
| 680 | $NH-C_2H_5$ | $NH_2$ | $CH=C(CN)_2$ | |
| 681 | $NH-nC_3H_7$ | $HN_2$ | $CH=C(CN)_2$ | |
| 682 | $NH-nC_4H_9$ | $NH_2$ | $CH=C(CN)_2$ | |
| 683 | $NH-secC_4H_9$ | $NH_2$ | $CH=C(CN)_2$ | |
| 684 | $NH-nC_6H_{13}$ | $NH_2$ | $CH=C(CN)_2$ | |
| 685 | $NH-nC_7H_{15}$ | $NH_2$ | $CH=C(CN)_2$ | |
| 686 | $NH-C_2H_4-OH$ | $NH_2$ | $CH=C(CN)_2$ | |
| 687 | $NH-C_2H_4-OCH_3$ | $NH_2$ | $CH=C(CN)_2$ | |
| 688 | NH-Cyclohexyl | $NH_2$ | $CH=C(CN)_2$ | |
| 689 | $NH-C_6H_5$ | $NH_2$ | $CH=C(CN)_2$ | |
| 690 | $NH-C_6H_4-(3)-CH_3$ | $NH_2$ | $CH=C(CN)_2$ | |
| 691 | $NH-CH_2-C\equiv CH$ | $NH_2$ | $CH=C(CN)_2$ | |
| 692 | $NH(CH_3)_2$ | $NH_2$ | $CH=C(CN)_2$ | |
| 693 | Pyrrolidino | $NH_2$ | $CH=C(CN)_2$ | |
| 694 | Piperidino | $NH_2$ | $CH=C(CN)_2$ | |
| 695 | Morpholino | $NH_2$ | $CH=C(CN)_2$ | |
| 696 | $NH-NH_2$ | $NH_2$ | $CH=C(CN)_2$ | |
| 697 | $NH-nC_3H_7$ | $NH_2$ | $CH=C(CN)\ (COOCH_3)$ | |
| 698 | $NH-nC_4H_9$ | $NH_2$ | $CH=C(CN)\ (COOCH_3)$ | |
| 699 | $NH-secC_4H_9$ | $NH_2$ | $CH=C(CN)\ (COOCH_3)$ | |
| 700 | $NH-nC_5H_{11}$ | $NH_2$ | $CH=C(CN)\ (COOC_2H_5)$ | |
| 701 | $N(C_2H_5)_2$ | $NH_2$ | $CH=C(CN)\ (COOC_2H_5)$ | |

| Bsp-Nr. | $R^3$ | $NR^1R^2$ | $R^4$ | Schmelzpunkt (°C) |
|---|---|---|---|---|
| 702 | $NH-CH_3$ | $NH_2$ | $CH=C(CN)(NO_2)$ | |
| 703 | $NH-isoC_3H_7$ | $NH_2$ | $CH=C(CN)(NO_2)$ | |
| 704 | (R/S)-NH-CH($CH_3$)-Cyclohexyl | $NH_2$ | CN | |
| 705 | $NH-(CH_2)_3-N(n-C_3H_7)_2$ | $NH_2$ | CN | |
| 706 | $NH-(CH_2)_3-N(iso-C_3H_7)_2$ | $NH_2$ | CN | |
| 707 | $NH-(+)-CH(CH_3)-C_6H_5$ | $NH_2$ | CN | |
| 708 | $NH-(-)-CH(CH_3)-C_6H_5$ | $NH_2$ | CN | |
| 709 | NH-Cyclobutyl | $NH_2$ | CN | |
| 710 | NH-Adamantyl | $NH_2$ | CN | |
| 711 | NH-Cyclononyl | $NH_2$ | CN | |
| 712 | NH-Cyclodecyl | $NH_2$ | CN | |
| 713 | NH-Methallyl | $NH_2$ | CN | |
| 714 | $N(isoC_3H_7)_2$ | $NH_2$ | CN | |
| 715 | N-Ethylpiperazino | $NH_2$ | CN | |
| 716 | Thiomorpholino | $NH_2$ | CN | |
| 717 | $NH-NH-C_2H_5$ | $NH_2$ | CN | |
| 718 | $NH-N(C_2H_5)_2$ | $NH_2$ | CN | |
| 719 | $N(CH_3)-NH-CH_3$ | $NH_2$ | CN | |
| 720 | Imidazol-1-yl | $NH_2$ | CN | |
| 721 | Pyrazol-1-yl | $NH_2$ | CN | |
| 722 | $NH-CH(CH_3)-(CH_2)_4CH_3$ | $NH_2$ | CN | |
| 723 | Pyrrolidino | $NH_2$ | CHO | 174-176 |

## Ansprüche

1. Aminothiazole der Formel I

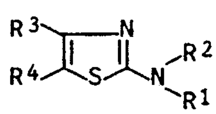

(I)

oder deren Tautomere, wobei

$R^1$ und $R^2$ jeweils Wasserstoff oder zusammen einen Rest der Formel

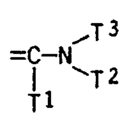

,

worin $T^1$ für Wasserstoff, $C_1$-$C_6$-Alkyl oder Phenyl und $T^2$ und $T^3$ gleich oder verschieden sind und unabhängig voneinander jeweils für $C_1$-$C_6$-Alkyl, $C_5$-$C_7$-Cycloalkyl oder Phenyl oder $T^2$ und $T^3$ zusammen mit dem sie verbindenden Stickstoffatom für einen 5- bis 7-gliedrigen gesättigten heterocyclischen Rest, der gegebenenfalls weitere Heteroatome enthält, stehen,

$R^3$ einen Rest der Formel

$$-S-(O)_n-X \quad ,$$

worin n für 0, 1 oder 2 und X für gegebenenfalls substituiertes $C_1$-$C_{20}$-Alkyl, $C_3$-$C_8$-Cycloalkyl, gegebenenfalls substituiertes $C_3$-$C_6$-Alkenyl, gegebenenfalls substituiertes $C_3$-$C_6$-Alkinyl, gegebenenfalls substituiertes Phenyl oder, wenn n die Bedeutung von 0 besitzt, auch für Wasserstoff stehen, oder einen Rest der Formel Y, der die Bedeutung $C_1$-$C_{20}$-Mono- oder Dialkylamino, worin die Alkylkette gegebenenfalls substituiert ist und durch ein oder mehrere Sauerstoffatome unterbrochen sein kann, $C_3$-$C_8$-Cycloalkylamino, Adamantylamino, $C_2$-$C_{12}$-Mono- oder Dialkenylamino, $C_3$-$C_{12}$-Alkinylamino, N-($C_1$-$C_5$-Alkyl)-N-phenylamino, Pyrrolidino, Piperidino, Morpholino, Thiomorpholino, Piperazino, N-($C_1$-$C_4$-Alkyl)-piperazino, Hexamethylenimino, Imidazol-1-yl, Pyrazol-1-yl, gegebenenfalls substituiertes Phenylamino, Pyridylamino, Thienylamino, Hydrazino, $C_1$-$C_4$-Mono- oder Dialkylhydrazino oder Phenylhydrazino besitzt, und

$R^4$ $C_1$-$C_6$-Alkanoyl, Benzoyl, Cyano oder einen Rest der Formel

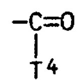

oder

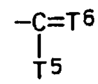

bedeuten, worin $T^4$ für Hydroxy, $C_1$-$C_6$-Alkoxy, Amino oder den obengenannten Rest Y, $T^5$ für Wasserstoff, $C_1$-$C_6$-Alkyl oder Phenyl und $T^6$ für den Rest einer methylenaktiven Verbindung, Hydroxyimino oder den Rest N-Q stehen, in dem Q die Bedeutung von $C_1$-$C_{20}$-Alkyl, das gegebenenfalls substituiert ist und durch ein oder mehrere Sauerstoffatome unterbrochen sein kann, gegebenenfalls substituiertem $C_3$-$C_6$-Alkenyl, gegebenenfalls substituiertem $C_3$-$C_6$-Alkinyl, gegebenenfalls substituiertem $C_3$-$C_{12}$-Cycloalkyl, gegebenenfalls substituiertem Phenyl, Pyridyl, $C_1$-$C_4$-Alkoxycarbonylmethyl, Amino, $C_1$-$C_4$-Dialkylamino oder Phenylamino besitzt, mit der Maßgabe, daß

a) wenn $R^1$ und $R^2$ jeweils Wasserstoff und $R^4$ Acetyl oder Ethoxycarbonyl bedeuten, $R^3$ nicht für den Rest der Formel

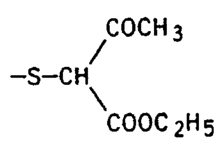

oder $-S-CH(COCH_3)_2$

stehen,

b) wenn $R^1$ und $R^2$ jeweils Wasserstoff und $R^4$ Cyano bedeuten, $R^3$ nicht für Methylthio steht, und

c) wenn $R^3$ für Piperidino oder Morpholino und $R^4$ für Cyano stehen, $R^1$ und $R^2$ nicht gleichzeitig Wasserstoff bedeuten.

**2.** Aminothiazole nach Anspruch 1, dadurch gekennzeichnet, daß
$R^1$ und $R^2$ jeweils Wasserstoff oder zusammen den Rest

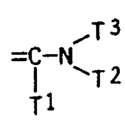

bedeuten, worin $T^1$ für Wasserstoff, $C_1$-$C_4$-Alkyl oder Phenyl und $T^2$ und $T^3$ unabhängig voneinander für $C_1$-$C_4$-Alkyl oder Phenyl, oder zusammen mit dem sie verbindenden Stickstoffatom für Pyrrolidino, Piperidino, Morpholino, Thiomorpholino, Piperazino, N-($C_1$-$C_4$-Alkyl)piperazino oder Hexamethylenimino stehen, und $R^3$ und $R^4$ jeweils die in Anspruch 1 genannte Bedeutung besitzen.

**3.** Verfahren zur Herstellung von Aminothiazolen gemäß Anspruch 1, dadurch gekennzeichnet, daß man Halogenaminothiazole der Formel II

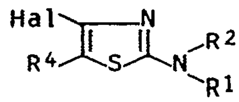

(II),

in der $R^1$, $R^2$ und $R^4$ jeweils die in Anspruch 1 genannte Bedeutung besitzen und Hal für Chlor oder Brom steht, mit einer Verbindung der Formel III

$$R^3\text{-}H \qquad (III),$$

in der $R^3$ die in Anspruch 1 genannte Bedeutung besitzt, umsetzt.